# EUROPEAN PATENT SPECIFICATION

(11) **EP 0 307 140 B1**
(45) Date of publication and mention of the grant of the patent: **02.03.1994**
(21) Application number: 88308125.9
(22) Date of filing: 01.09.1988
(51) Int. Cl.: C07D 487/08, A61K 31/40

(54) **Oxadiazolyl-azabicycloheptanes for the treatment of senile dementia**
Oxadiazolyl-azabicycloheptane zur Behandlung von seniler Demenz
Oxadiazolyl-azabicycloheptanes pour le traitement de la démence sénile

(30) Priority: 10.09.1987 GB 8721344; 20.07.1988 GB 8817310
(43) Date of publication of application: 15.03.1989
(73) Proprietor: MERCK SHARP & DOHME LTD., Hoddesdon Hertfordshire EN11 9NU (GB)
(72) Inventor: Baker, Raymond, Much Hadham Hertfordshire (GB); Saunders, John, Bishops Stortford Hertfordshire (GB); Street, Leslie J., Harlow Essex (GB)
(74) Representative: Cole, William Gwyn

(56) References cited:
- EP-A- 0 239 309
- EP-A- 0 261 763
- THE JOURNAL OF ORGANIC CHEMISTRY, vol.34, 1969 D:O Spry et al, pages 3674-3676

## Description

The present invention relates to a class of substituted oxadiazole compounds which stimulate central muscarinic acetylcholine receptors and therefore are useful in the treatment of neurological and mental illnesses whose clinical manifestations are due to cholinergic deficiency. Such diseases include presenile and senile dementia (also known as Alzheimer's disease and senile dementia of the Alzheimer type respectively), Huntington's chorea, tardive dyskinesia, hyperkinesia, mania and Tourette Syndrome. Alzheimer's disease, the most common dementing illness, is a slowly progressive neurological disorder characterised by marked deficits in cognitive functions including memory, attention, language and visual perception capabilities. The compounds of this invention are also useful analgesic agents and therefore useful in the treatment of severe painful conditions such as rheumatism, arthritis, and terminal illness.

Compounds capable of enhancing muscarinic cholinergic transmission in the cortex should be beneficial in reversing the cholinergic deficiency in Alzheimer's disease and other diseases related to cholinergic dysfunction. However, most muscarinic agonists, including acetylcholine itself, are quaternary ammonium compounds incapable of penetrating the blood-brain barrier to any clinically significant extent following peripheral (e.g. oral) administration. Such agents fail to stimulate the desired central sites but instead induce undesired side-effects mediated exclusively by peripherally-located muscarinic acetylcholine receptors.

The oxadiazole compounds of the present invention are potent muscarinic agonists but, being tertiary amines with physiochemical properties (lipophilicity and pKa) consistent with CNS penetrability, can stimulate those central sites implicated in neurodegenerative disorders. It is believed that the enhancement of cholinergic transmission demonstrated by the compounds of this invention is achieved either directly by stimulating postsynaptic receptors, or indirectly by potentiating acetylcholine release.

EP-A-0261763, which was published on 30 March 1988, describes a class of compounds which includes oxadiazoles bearing a particular unsubstituted exo-1-azabicyclo[2.2.1]heptane substituent; these compounds are stated to be of potential use in the treatment and/or prophylaxis of dementia in mammals. This document does not, however, disclose exo-1-azabicyclo[2.2.1]heptane-substituted oxadiazoles in which the azabicyclic substituent is itself substituted.

In addition, EP-A-0239309, which was published on 30 September 1987, describes a class of oxadiazole compounds which are stated to be potent muscarinic agonists. These oxadiazoles are substituted on one of the ring carbon atoms thereof with a non-aromatic azacyclic or azabicyclic ring system; and substituted on the other ring carbon atom with a substituent of low lipophilicity.
EP-A-0239309 specifically discloses 3-[5-(3-amino-1,2,4-oxadiazol)-yl]-1-azabicyclo[2.2.1]-heptane; it will be noted, however, that the azabicyclic moiety of this latter compound is unsubstituted. Moreover, EP-A-0239309 generically discloses oxadiazoles in which the azabicyclic ring system is a 1-azabicyclo[2.2.1]heptane ring system optionally substituted with methyl or hydroxy. Nevertheless, none of the oxadiazole compounds specifically disclosed in EP-A-0239309 possesses a 1-azabicyclo[2.2.1]heptane substituent which is itself substituted.

The present invention provides an oxadiazole represented by structural formula I: 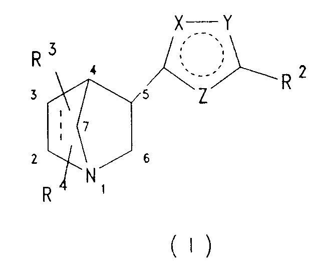
or a salt or prodrug thereof; wherein
one of X, Y or Z is an oxygen atom and the other two are nitrogen atoms, and the dotted circle represents aromaticity (two double bonds);
R² represents a substituent of low lipophilicity selected from hydrogen, halogen -CF₃, -OR⁷, -NR⁷R⁸, -NHOR⁷, -NHNH₂, -CN, -CO₂R⁷, -CON⁷R⁸, C₂₋₅ alkenyl, C₂₋₅ alkynyl, C₁₋₂ alkyl, or C₁₋₂ alkyl substituted with -OR⁷, -NR⁷R⁸, -SR⁷, -CO₂R⁷, -CONR⁷R⁸ or halogen;
the broken line represents an optional chemical bond; and
the substituents R³ and R⁴ may be present at any position, including the point of attachment to the oxadiazole ring, and R³ represents halo, C₁₋₄ alkoxy, carboxy, -NR⁷R⁸, C₂₋₄ alkyl, C₁₋₄ alkyl substituted with hydroxy or C₁₋₄ alkoxy, or methyl or hydroxy in the 3-, 4- or 5-position; and R⁴ represents hydrogen, halo, C₁₋₄ alkoxy, hydroxy, carboxy, -NR⁷R⁸, C₁₋₄ alkyl, or C₁₋₄ alkyl substituted with hydroxy or C₁₋₄ alkoxy; or R³ and R⁴ together represent carbonyl; and
R⁷ and R⁸ independently represent hydrogen or C₁₋₂ alkyl.

It will be appreciated that the nitrogen atom in the azabicycloheptane ring will carry a lone pair of electrons.

Preferably the oxadiazole ring is a 1,2,4-oxadiazole.

Suitably the group R⁴ is hydrogen or methyl; and R³ is C₁₋₄ alkoxy, halo, -NR⁷R⁸, hydroxy(C₁₋₄)alkyl or C₁₋₄ alkoxymethyl, preferably methoxy, fluoro, amino, methoxymethyl or hydroxymethyl. Preferably R⁴ is hydrogen.

The term "low lipophilicity" is intended to indicate that the group R₂ has a Rekker f value (hydrophobic fragment constant; see R. F. Rekker, "The Hydrophobic Fragmental Constant", Elsevier, 1977) of not greater than 1.5. For example, the methyl group has a value of 0.7 and the ethyl group a value of 1.26.

Thus the substituent of low lipophilicity represented by the group R² in formula I may be hydrogen, halogen, -CF₃, -OR⁷, -NR⁷R⁸, -NHOR⁷, -NHNH₂, -CN, -CO₂R⁷, -CONR⁷R⁸, C₂₋₅ alkenyl, C₂₋₅ alkynyl, C₁₋₂ alkyl, or C₁₋₂ alkyl substituted with -OR⁷, -NR⁷R⁸, -SR⁷, -CO₂R⁷, -CONR⁷R⁸ or halogen; wherein R⁷ and R⁸ are as defined with respect to formula I above.

Preferably the substituent of low lipophilicity is hydrogen, halogen, -CF₃, -OR⁷, -NR⁷R⁸, -NHNH₂, -CN, -CO₂R⁷, -CONR⁷R⁸, C₂₋₃ alkenyl, C₂₋₃ alkynyl, C₁₋₂ alkyl, or C₁₋₂ alkyl substituted with -OR⁷, -NR⁷R⁸, -SR⁷, -CO₂R⁷, -CONR⁷R⁸ or halogen. Particular values of R² are hydrogen, methyl, amino, dimethylamino, methoxycarbonyl and ethoxycarbonyl. A preferred group R² is amino.

As used herein, the terms "alkyl" and "alkoxy" include straight chain alkyl and, where the alkyl group is of 3 or more carbons, branched chain alkyl and cycloalkyl. The terms "alkenyl" and "alkynyl" encompass both straight and branched chain. The term "halo" or "halogen" means fluoro, chloro or bromo.

One group of prodrugs of compounds of this invention have a substituent on the oxadiazole ring which is hydrolysable in vivo to an amino group.

Groups which are hydrolysable in vivo to an amino group on the compounds of this invention may be readily ascertained by administering the compound to a human or animal and detecting, by conventional analytical techniques, the presence of the corresponding compound having an amino substituent in the urine of a human or animal. Examples of such groups include, for example, amido and urethane substituents, in particular a group of formula -NH.Q, wherein Q represents CHO, COR or CO₂R, and R represents an optionally substituted hydrocarbon group.

In this context, the hydrocarbon group R includes groups having up to 20 carbon atoms, suitably up to 10 carbon atoms, conveniently up to 6 carbon atoms. Suitable hydrocarbon groups include C₁₋₆ alkyl, C₂₋₆ alkenyl, C₂₋₆ alkynyl, C₃₋₇ cycloalkyl, C₃₋₇ cycloalkyl(C₁₋₆)alkyl, aryl, and aryl(C₁₋₆)alkyl. The alkyl group R may be straight or branched chain and may contain, for example, up to 12 carbon atoms, suitably from 1 to 6 carbon atoms. In particular the group may be substituted methyl, ethyl, n- or iso-propyl, n-, sec-, iso- or tert-butyl, n- or iso-heptyl, or n- or iso-octyl. Suitable cycloalkyl groups include cyclopentyl and cyclohexyl. The aryl group R includes phenyl and naphthyl optionally substituted with up to five, preferably up to three, substituent groups.

One sub-class of compounds within the scope of the present invention is represented by formula IIA: 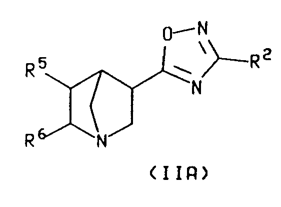
wherein R² is as defined above, R⁵ represents C₁₋₄ alkyl, halo, -NR⁷R⁸, C₁₋₄ alkoxy, hydroxy(C₁₋₄)alkyl, C₁₋₄ alkoxy(C₁₋₄)alkyl or hydroxy, and R⁶ represents hydrogen, C₁₋₄ alkyl, C₁₋₄ alkoxy or hydroxy; in particular wherein R² represents C₁₋₂ alkyl, amino, dimethylamino or C₁₋₃ alkoxycarbonyl, R⁵ represents C₁₋₃ alkyl, hydroxy, C₁₋₃ alkoxy, fluoro, hydroxymethyl, methoxymethyl or amino, and R⁶ represents hydrogen.

Another sub-class of compounds within the scope of this invention is represented by formula IIB: 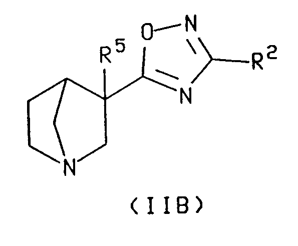
wherein R² and R⁵ are as defined with respect to formula IIA above; in particular wherein R² represents C₁₋₃ alkyl and R⁵ represents hydroxy.

A further sub-class of compounds within the scope of this invention is represented by formula IIC: 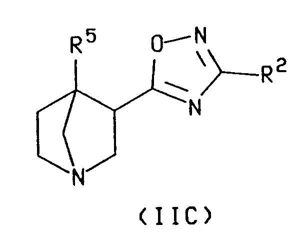
wherein R² and R⁵ are as defined with respect to formula IIA above; in particular wherein R² and R⁵ each represents C₁₋₃ alkyl.

Specific compounds within the scope of the present invention include:
3-[5-(3-methyl-1,2,4-oxadiazol)-yl]-1-azabicyclo[2.2.1]heptan-5-ol;
3-[5-(3-methyl-1,2,4-oxadiazol)-yl]-1-azabicyclo[2.2.1]heptan-3-ol;
3-[5-(3-methyl-1,2,4-oxadiazol)-yl]-5-ethyl-1-azabicyclo[2.2.1]heptane;
3-[5-(3-methyl-1,2,4-oxadiazol)-yl]-6-methyl-1-azabicyclo[2.2.1]heptane;
3-[5-(3-amino-1,2,4-oxadiazol)-yl]-1-azabicyclo[2.2.1]heptan-5-ol;
3-[5-(3-methyl-1,2,4-oxadiazol)-yl]-5-fluoro-1-azabicyclo[2.2.1]heptane;
5-[5-(3-methyl-1,2,4-oxadiazol)-yl]-3-methoxymethyl-1-azabicyclo[2.2.1]heptane;
5-[5-(3-methyl-1,2,4-oxadiazol)-yl]-3-methyl-1-azabicyclo[2.2.1]heptane;
3-[5-(3-methyl-1,2,4-oxadiazol)-yl]-5-hydroxymethyl-1-azabicyclo[2.2.1]heptane;
3-[5-(3-dimethylamino-1,2,4-oxadiazol)-yl]-1-azabicyclo[2.2.1]heptan-5-ol;
3-[5-(3-dimethylamino-1,2,4-oxadiazol)-yl]-5-methoxy-1-azabicyclo[2.2.1]heptane;
3-[5-(3-dimethylamino-1,2,4-oxadiazol)-yl]-5-methyl-1-azabicyclo[2.2.1]heptan-5-ol;
3-[5-(3-dimethylamino-1,2,4-oxadiazol)-yl]-5-amino-1-azabicyclo[2.2.1]heptane;
3-[5-(3-methyl-1,2,4-oxadiazol)-yl]-4-methyl-1-azabicyclo[2.2.1]heptane;
and salts and prodrugs thereof.

The compounds of this invention all have more than one asymmetric centre, and can therefore exist as both enantiomers and diastereoisomers. In particular, they can exist as exo and endo isomers. It is to be understood that the invention covers all such isomers and mixtures thereof.

Also included within the scope of the present invention are salts of the novel compounds. It will be appreciated that salts of the compounds for use in medicine will be non-toxic pharmaceutically acceptable salts. Other salts may, however, be useful in the preparation of the compounds of the invention or their non-toxic pharmaceutically acceptable salts. Acid addition salts, for example, may be formed by mixing a solution of the compound with a solution of a pharmaceutically acceptable non-toxic acid such as hydrochloric acid, fumaric acid, maleic acid, succinic acid, acetic acid, citric acid, tartaric acid, carbonic acid or phosphoric acid. Where the novel compound carries a carboxylic acid group the invention also contemplates salts thereof, preferably non-toxic pharmaceutically acceptable salts thereof, such as the sodium, potassium or calcium salts thereof.

Salts of amine groups may also comprise the quaternary ammonium salts in which the amino nitrogen atom carries an alkyl, alkenyl, alkynyl or aralkyl group. Such quaternary ammonium derivatives penetrate poorly into the central nervous system and are therefore useful as peripherally selective muscarinic agents, agents to reduce gastric acid secretion, agents to block the muscarinic actions of acetylcholinesterase inhibitors in the treatment of myasthenia gravis and as agents to co-administer with muscarinic agonists in Alzheimer's disease.

It is believed that those compounds of the invention which directly stimulate post-synaptic receptors are particularly useful as analgesic agents.

This invention therefore also provides a pharmaceutical composition comprising a compound of the invention and a pharmaceutically acceptable carrier.

It may, where appropriate, be advantageous, in order to reduce unwanted peripherally mediated side-effects, to incorporate into the composition a peripherally acting cholinergic antagonist (or anti-muscarinic agent). Thus the compounds of the invention may advantageously be administered together with a peripheral cholinergic antagonist such as N-methylscopolamine, N-methylatropine, propantheline, methantheline or glycopyrrolate.

The compounds of the invention can be administered orally, parenterally or rectally at a daily dose of about 0.01 to 10 mg/kg of body weight, preferably about 0.1 to 1 mg/kg, and may be administered on a regimen of 1-4 times a day. When a cholinergic antagonist is administered, it is incorporated at its conventional dose.

The pharmaceutical formulations of this invention preferably are in unit dosage forms such as tablets, pills, capsules, powders, granules, sterile parenteral solutions or suspensions, or suppositories for oral, parenteral or rectal administration. For preparing solid compositions such as tablets, the principal active ingredient is mixed with a pharmaceutical carrier, e.g. conventional tabletting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups and flavoured emulsions with edible oils such as cottonseed oil, sesame oil, coconut oil and peanut oil, as well as elixirs and similar pharmaceutical vehicles. Suitable dispersing or suspending agents for aqueous suspension include synthetic and natural gums such as tragacanth, acacia, alginate, dextran, sodium carboxymethylcellulose, methylcellulose, polyvinyl-pyrrolidone and gelatin.

The compounds of this invention may be prepared by a process which comprises reacting a reactive derivative of a carboxylic acid of formula R^{a}-CO₂H with either a compound of formula IIIA or a compound of formula IIIB or a salt thereof: 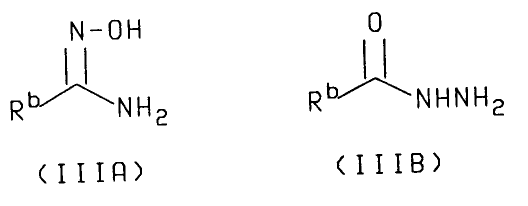
wherein one of R^{a} and R^{b} is a substituted 1-azabicyclo[2.2.1]heptane ring, and the other is a group of low lipophilicity.

Suitable reactive derivatives of the acid R^{a}-CO₂H include esters, for example C₁₋₄ alkyl esters; thioesters, for example pyridylthioesters; acid anhydrides, for example (R^{a}CO)₂O; acid halides, for example acid chlorides; orthoesters; and primary, secondary and tertiary amides.

When the compound of formula IIIA is employed the product of the reaction is a 1,2,4-oxadiazole. It will be appreciated that the compound IIIA can also be considered as the alternative tautomeric form: 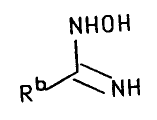
A 3-substituted 1,2,4-oxadiazol-5-yl compound is produced if R^{a} represents the azabicycloheptane group and R^{b} in formula IIIA represents the substituent of low lipophilicity. In this case, a preferred reactive derivative of the acid R^{a}CO₂H is a C₁₋₄ alkyl ester. The reaction is conveniently carried out in tetrahydrofuran, dimethylformamide or a lower alkanol such as ethanol, propanol or isopropanol at about 20° to 100°C for about 1 to 6 hours.

A 5-substituted 1,2,4-oxadiazol-3-yl compound is produced by the process of this invention when R^{a} represents the substituent of low lipophilicity and R^{b} represents the azabicycloheptane group. For this reaction a suitable reactive derivative is the acid chloride or the acid anhydride (R^{a}CO)₂O. The reaction may be carried out by treating compound IIIA, in the cold, e.g. from about -5° to +10°C, with the reactive derivative, followed by heating at about 80° to 120°C for about 1 to 6 hours.

When the compound of formula IIIB is employed, the product of the process of this invention is a 1,3,4-oxadiazole. In this case, a preferred reactive derivative of the acid R^{a}CO₂H is an orthoester of formula R^{a}C(OR⁹)₃ where R⁹ represents C₁₋₃ alkyl. The process is conveniently effected by heating the hydrazide IIIB with the orthoester in a solvent such as methanol at reflux temperature for about 2 to 8 hours. An intermediate of formula R^{b}.CO.NH.N=C(R^{a})OR⁹ may be isolated by evaporation of the solvent. The intermediate is then treated with a strong base such as potassium t-butoxide or 1,8-diazabicyclo[5.4.0]undec-7-ene in butanol for about 10 to 24 hours at about 90° to 150°C.

After the above process is complete, one substituent of low lipophilicity can be converted to another. For example an amino group may be converted to chloro, or hydrazo, -NHNH₂, via the intermediacy of diazonium, -N₂⁺. Similarly, a chloro substituent may be converted to methoxy by reaction with a nucleophile such as methoxide; and alkoxycarbonyl groups may be converted, via carboxy, to an amino substituent, -NH₂.

The reactive derivatives of the compound R^{a}CO₂H may, for example, be prepared by cyclisation of a compound of formula IV: 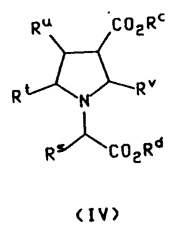
wherein R^{s}, R^{t}, R^{u} and R^{v} represent hydrogen, carboxylic acid ester, or a substituent R³ or R⁴ as defined above, or a group which is convertible thereto; at least one of R^{s}, R^{t}, R^{u} and R^{v} being other than hydrogen; and
R^{c} and R^{d} are hydrocarbon groups, in particular C₁₋₆ alkyl, such as methyl or ethyl; to produce a compound of formula V: 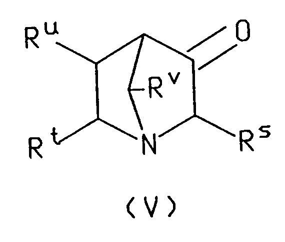
or a ketal thereof; and optionally converting the carbonyl group in compound V or the ketal thereof, or any of the groups R^{s}, R^{t}, R^{u} and R^{v}, to a substituent group R³ or R⁴ or to a -CO₂H group or reactive derivative thereof.

The intermediate of formula V is a novel compound and forms a further aspect of this invention. It provides a valuable route to substituted 1-azabicyclo[2.2.1]heptanes used in the process of the present invention.

The cyclisation of compound IV is carried out in the presence of a strong base, such as potassium t-butoxide, followed by acidification, for example by concentrated hydrochloric acid. This reaction causes the condensation of the groups CO₂R^{a} and CO₂R^{b}. When one of the groups R^{s}, R^{t}, R^{u} and R^{v} represents an ester group, this reaction will liberate the free acid. For ease of isolation it is preferable to esterify the acid in situ, for example by treatment with methanol and hydrochloric acid.

The carboxylic acid group and thence its reactive derivative, which is employed to react with the oxadiazole moiety, may be generated from any of the substituent groups R^{s}, R^{t}, R^{u} or R^{v} initially present in compound IV. In this way, by selection of one such group as an ester group, the point of attachment to the oxadiazole can be determined.

Alternatively the ketone group which is generated in the intermediate V may be converted to a carboxylic acid group, for example by reaction with an alkali metal cyanide such as sodium cyanide, to produce a compound VI: 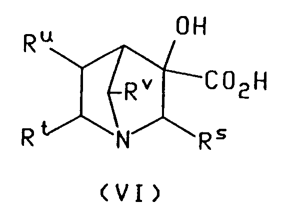
The hydroxy group in compound VI may then be converted to a group R³ or R⁴ to produce a substituent at the same position as the point of attachment to the oxadiazole ring.

In any of the above reactions it may be necessary and/or desirable to protect any sensitive groups in the compounds. For example, if R^{a} and/or R^{b} include amino, carboxy, hydroxy or thiol groups, these may be protected in conventional manner. Thus, suitable protecting groups for hydroxy groups include silyl groups such as trimethylsilyl or t-butyldimethylsilyl, and etherifying groups such as tetrahydropyranyl; and for amino groups include benzyloxycarbonyl and t-butyloxycarbonyl. Carboxy groups are preferably protected in a reduced form such as in the form of their corresponding protected alcohols, which may be subsequently oxidised to give the desired carboxy group. Thiol groups may be protected by disulphide formation, either with the thiol itself or with another thiol to form a mixed disulphide. The protecting groups may be removed at any convenient stage in the synthesis of the desired compound according to conventional techniques.

The following Examples illustrate the preparation of compounds according to the invention. Each of the compounds of the Examples demonstrates an affinity for the muscarinic receptor, having an IC₅₀ (concentration required to displace 50% of specific [³H]-N-methylscopolamine binding from rat cortical membrane preparations) significantly lower than 100µM. Agonist behaviour and penetrability into the central nervous system of the compounds of the Examples were assessed in a rat behavioural model by measuring the ability of the compound under test to elicit a mouth movement response and/or a hypothermic response characteristic of centrally-active muscarinic agonists (see Salamone et al., Psychopharm., 1986, 88, 467). In this model, the compounds of all the Examples were active at doses of 10 mg/kg or less.

In the Examples, all temperatures are in °C; THF is tetrahydrofuran; and ether is diethyl ether.

### EXAMPLE 1

### exo - 3[5-(3-Methyl-1,2,4-oxadiazol)yl]-1-azabicyclo [2.2.1]heptan-3-ol hydrochloride

### (a) 1-Benzyl-3-carbomethoxypyrrolidine

This was prepared from 1-benzyl-3-carbomethoxy-5-pyrrolidinone by the procedure described by Kornet et al, J. Org. Chem., 1968, 33 3637.

### (b) 1-Methoxycarbonylmethyl-3-methoxycarbonylpyrrolidine.

A solution of 1-benzyl-3-carbomethoxypyrrolidine (50g, 228 mmol) in methanol (300 ml) was subjected to hydrogenolysis over Pd(OH)₂ (6g) on a Parr shaker for 7 hours. The suspension was filtered through celite and the solvent removed under vacuum to give 3-carbomethoxypyrrolidine (30 g) as a clear liquid. This amine (29 g, 225 mmol) was added to a rapidly stirred suspension of potassium carbonate (64 g) in xylene (350 ml) at 130°C. After 0.25 hours a solution of methylbromoacetate (35 g, 230 mmol) in xylene (100 ml) was added dropwise and the mixture heated under reflux for 2 hours. The xylene solution was decanted from the inorganic residue which was taken up into water (200 ml) and extracted with dichloromethane (3 x 250 ml).

The combined organics were dried (sodium sulphate) and evaporated to afford the title compound (43 g) as a yellow liquid, δ (60MHz CDCl₃) 2.10 - 3.30 (7H, m, 3 x CH₂ and 1 x CH); 3.37 (2H, s, CH₂ - CO₂Me) and 3.72 (6H, s, 2 x CO₂Me).

### (c) 1-Azabicyclo[2.2.1]heptan-3-one

A solution of 1-methoxycarbonylmethyl-3-methoxycarbonylpyrrolidine (5g, 24.9 mmol) in toluene (75 ml) was added dropwise, over a 3 hour period, to a rapidly stirred solution of potassium-t-butoxide (8 g, 71 mmol) in toluene (250 ml) at 130°C. The mixture was heated under reflux for 4 hours, cooled to room temperature, and concentrated hydrochloric acid (75 ml) added dropwise and stirred for 0.25 hour. The separated toluene solution was extracted with concentrated hydrochloric acid (3 x 50 ml) and the combined aqueous extracts heated under reflux for 16 hours. The solvent was reduced to half volume under vacuum, basified to PH > 10 with potassium carbonate and extracted with chloroform (6 x 100 mls). The material isolated from the organic extracts was chromatographed on silica in dichloro methane-methanol (90 : 10) to give 1-azabicyclo-[2.2.1]heptan-3-one (1.2 g) as a yellow oil, δ (360 MHz, CDCl₃) 1.75-1.80 (1H, m, 0.5 x CH₂); 2.06-2.12 (1H, m. 0.5 x CH₂); 2.70-2.81 (4H, m, 2 x CH₂-N), and 3.00-3.12 (3H, m, CH₂-N and CH).

### (d) exo-3-Carbomethoxy-1-azabicyclo[2.2.1]heptan-3-ol

The hydrochloride salt of 1-azabicyclo[2.2.1]heptan-3-one was prepared by addition of ethereal hydrogen chloride to a solution of the compound in methanol. A solution of sodium cyanide(0.7 g, 14.3 mmol) in water (4 ml) was added dropwise to a solution of 1-azabicyclo-[2.2.1]heptan-3-one hydrochloride (1.7 g, 11.5 mmol) in water (5 ml) at -5°C. The mixture was stirred at 0°C tor 1 hour and the precipitated cyanohydrin filtered and washed with cold water The solid was taken up into concentrated hydrochloric acid (15 ml) and stirred for 24 hours at room temperature. The residue obtained after removal of the solvent and drying was dissolved in a saturated solution of hydrogen chloride in methanol (100 ml) and stirred at room temperature for 16 hours. The solvent was removed under vacuo, the residue taken up into water (25 ml), basified to pH 10 with potassium carbonate and extracted with dichloromethane (8 x 50 ml). The combined extracts were dried and evaporated to give the title compound (1.3 g), m/e 171 (M⁺); δ (360 MHz, CDCl₃) 1.39-1.48 (1H, m, 0.5 x CH₂); 2.16-2.21 (1H, m, 0.5 x CH₂); 2.40-2.50 (2H, m, CH₂-N); 2.66-2.72 (2H, m, CH and 0.5 x CH₂-N); 3.22-3.26 (1H, m, 0.5 x CH₂-N); 3.83 (3H, s, CO₂me).

### (e) exo-3[5-(3-Methyl-1,2,4-oxadiazol)yl]-1-azabicyclo [2.2.1]heptan-3-ol hydrochloride.

Activated molecular sieves (Type 4A, 0.5 g) were added to a stirred solution of acetamide oxime (0.4 g, 5.4 mmol) in anhydrous tetrahydrofuran (50 ml) under nitrogen. After 0.5 hour, sodium hydride (0.14 g of a 80% dispersion in oil, 4.6 mmol) was added and the solution stirred for a further 0.5 hour. A solution of exo-3-carbomethoxy-1-azabicyclo[2.2.1]heptan-3-ol (0.4 g, 2.34 mmol) in tetrahydrofuran (20 ml) was then added and the mixture stirred under reflux for 2 hours. The mixture was cooled to room temperature, quenched with water (20 ml) and extracted with dichloromethane (5 x 100 ml). The combined extracts were dried (sodium sulphate) the solvent evaporated and the residue chromatographed through alumina using dichloromethane-methanol (95 : 5) as eluant to give the title oxadiazole (0.2 g) as a crystalline solid. The product was further purified as the hydrochloride salt, m.p. 225-227°C (isopropanol); (Found C, 46.33; H, 6.07; N, 17.81 C₉H₁₃N₃O₂.HCl requires C, 46.65; H, 6.05; N,18.14%; m/e 195 (M⁺ for free base); δ (360 MHz, CDCl₃) 1.46-1.56 (1H, m, 0.5 x CH₂); 2.12-2.24 (1H, m, 0.5 x CH₂); 2.45 (3H, s, Me); 3.26-3.34 (1H, m, 0.5 x CH₂-N); 3.35 (1H, d, J = 4.75 Hz, CH-bridge-head); 3.44-3.60 (2H, m, 2 x 0.5 x CH₂-N); 3.75 (1H, dd, J = 2.8 and 13 Hz, 0.5 x CH₂-N); 3.88-3.90 (1H, m, 0.5 x CH₂N); 4.1 (1H, dd, J = 2.48 and 13 Hz, 0.5 x CH₂-N).

### EXAMPLE 2

3[5-(3-Methyl-1,2,4-oxadiazol)yl]-1-azabicyclo[2.2.1]heptan-5-ols

### (a) trans-3,4-Dicarbomethoxypyrrolidine

This was prepared from glycine by the procedure reported by Joucla et al, (J. Chem. Soc., Chem. Commun, 1985, 1566).

### (b) 1-Methoxycarbonylmethyl-trans-3,4-dicarbo methoxypyrrolidine

A solution of trans-3,4-dicarbomethoxypyrrolidine (4.1 g, 22 mmol) in xylene (30 ml) was added to a rapidly stirred suspension of potassium carbonate (7 g) in xylene (150 ml) at 120°C. After 0.25 hours a solution of methylbromoacetate (3.45 g, 22.5 mmol) in xylene (30 ml) was added dropwise and the mixture stirred and heated under reflux for 2 hours. The solution was then decanted from the inorganic residue which was taken up into water (100 ml) and extracted with dichloromethane (3 x 150 ml). The combined organics were dried (sodium sulphate) and evaporated to afford the title compound as a yellow liquid (6 g); δ (360 MHz, CDCl₃) 2.96-3.11 (4H, m, 2 x CH₂-N); 3.34 (2H, ABq, J = 16.5Hz, CH₂-CO₂me); 3.46-3.52 (2H, m, 2 x CH) and 3.74 (9H, s, 3 x CO₂me).

### (c) 3-Carbomethoxy-5,5-dimethoxy-1-azabicyclo [2.2.1]-heptane

A solution of 1-methoxycarbonylmethyl-trans-3,4-dicarbomethoxypyrrolidine (5 g, 19.31 mmol) in toluene (75 ml) was added dropwise over a 3 hour period, to a rapidly stirred solution of potassium t-butoxide (9 g, 80 mmol) in toluene (250 ml) at 130°C. The mixture was heated under reflux for 4 hours, cooled to room temperature, and concentrated hydrochloric acid (75 ml) added dropwise and stirred for 0.25 hour. The separated organic phase was extracted with concentrated hydrochloric acid (3 x 50 ml) and the combined aqueous extracts heated under reflux for 16 hours. The solvent was removed in vacuo, the residue dried and taken up into a saturated solution of hydrogen chloride in methanol (150 ml). The mixture was stirred at room temperature for 24 hours, the solvent removed in vacuo, water (50 ml) added and basified to pH>10 with potassium carbonate. The solution was extracted with dichloromethane (5 x 150 ml) and the combined extracts dried (sodium sulphate) and evaporated. The residue was purified by chromatography on silica-gel in dichloromethane-methanol (93 : 7) to give the title azanorborane as a yellow liquid (0.5 g), and as a single isomer δ (360 MHz, CDCl₃) 2.44 (1H, dd, J=9.8, 3Hz, 0.5 x CH₂); 2.63 (1H, dd, J=12.7, 3Hz, 0.5 x CH₂; 2.77 (1H, d, J=12.7Hz, 0.5 x CH₂); 2.80-3.10 (5H, m, 2 x CH and 1.5 x CH₂); 3.11 (3H, s, OMe); 3.24 (3H, s, OMe); 3.71 (3H,s,CO₂Me).

### (d) 3[5-(3-Methyl-1,2,4-oxadiazol)yl]-5,5-dimethoxy-1-azabicyclo[2.2.1]-heptane.

Activated molecular series (Type 4A, 1 g) were added to a stirred solution of acetamide oxime (0.6 g, 8.1 mmol) in anhydrous tetrahydrofuran (50 ml) under nitrogen. After 0.5 hour, sodium hydride (0.2 g of a 80% dispersion in oil, 6.7 mmol) was added and the solution stirred for a further 0.5 hour. A solution of exo-3-carbomethoxy-5,5-dimethoxy-1-azabicyclo[2.2.1]-heptane (0.5 g, 2.3 mmol) in tetrahydrofuran (20 ml) was then added and the mixture heated at reflux for 6 hours. The mixture was cooled to room temperature diluted with water (15 ml) and extracted with dichloromethane (5 x 100 ml). The combined extracts were dried (sodium sulphate), the solvent evaporated and the residue chromatographed through silica-gel using dichloro-methane-methanol (95:5) as eluant to afford a clear oil (0.26 g), δ (360 MHz, CDCl₃) 2.38 (3H, s, Me); 2.43 (1H, dd, J=12.7, 3 Hz, 0.5 x CH₂); 2.73 (1H, dd, J=10, 3.2 Hz, 0.5 x CH₂); 2.95 (1H, d, J=12.8 Hz 0.5 x CH₂); 2.99 (1H, s, CH); 3.10 (1H, d, J=12.8 Hz, 0.5 x CH₂); 3.07-3.22 (2H, m, CH₂); 3.22 (3H, s, O Me); 3.27(3H, s, O Me); 3.47-3.50 (1H, m, CH).

### (e) 3[5-(3-Methyl-1,2,4-oxadiazol)yl]-1-aza bicyclo[2.2.1]-heptan-5-one.

A solution of 3[5-(3-methyl-1,2,4-oxadiazol)yl]-5,5-dimethoxy-1-azabicyclo[2.2.1]heptane (0.26 g, 1.26 mmol) in perchloric acid (2.5 ml) was stirred at 85°C for 16 hours. The mixture was diluted with water (10 ml), neutralised with sodium carbonate and extracted with dichloromethane (4 x 50 ml). The combined extracts were dried (sodium sulphate) and evaporated and the residue chromatographed on silica-gel using dichloromethane-methanol (93 : 7) as eluant to give the title ketone as a mixture of two isomers (0.2 g), δ (360 MHz, CDCl₃), 2.35 and 2.39 (3H, s, Me); 2.89-3.94 (8H, m 3 x CH₂ and 2 x CH).

### (f) 3[5-(3-Methyl-1,2,4-oxadiazol)yl]-1-azabicyclo-[2.2.1]heptan-5-ols

Sodium borohydride (30 mg, 0.8 mmol) was added to a solution of 3-[5(3-methyl-1,2,4-oxadiazol)yl]-1-azabicyclo[2.2.1]heptan-5-one (0.2 g, 1 mmol) in ethanol (20 ml), at 0°C. The mixture was stirred at 0°C for 0.5 hour and then at room temperature for 0.5 hour. Excess borohydride was destroyed by addition of 2M hydrochloric acid and the solvent removed under reduced pressure. The residue was taken up into water (10 ml) and basified to pH 10 with potassium carbonate. Extraction with dichloromethane (5 x 50 ml), drying (sodium sulphate) and evaporation of solvent gave a crude product which was chromatographed through silica-gel using dichloromethane-methanol (90:10) to afford two separated components in a ratio of 3:1. The less polar, major isomer, Isomer A, was isolated as a white crystalline solid, m.p. 136-139°C (isopropylalcohol-ether). (Found C, 55.02; H, 6.69; N, 21.16. C₉H₁₃N₃O₂ requires C, 55.37, H, 6.71, N, 21.52%); δ (360 MHz, CDCl₃) 2.17 (1H, dt, J = 3.6, 13 Hz, 0.5 x CH₂); 2.38 (3H, s, CH₃); 2.68 (1H, broad d, J = 10 Hz, 0.5 x CH₂); 2.79 (1H, dd, J = 3.6, 10 Hz, 0.5 x CH₂; 2.95 (1H, d, J = 4.4 Hz, -CH); 3.11-3.22 (3H, m, 0.5 x CH₂ and CH₂); 3.88 (1H, dd, J = 6.4 Hz, -CH-oxadiazole); 4.50-4.55 (1H, m, -CH-OH).

The more polar isomer, Isomer B, was isolated as a crystalline solid, m.p. 171 -174°C (isopropylacoholether δ (360 MHz, CDCl₃) 2.30 (1H, dt, J = 3.8, 13 Hz, 0.5 x CH₂); 2.37 (3H, s, CH₃); 2.68 (1H, dd, J = 3.5, 10 Hz, 0.5 x CH₂); 2.75 (1H, dd, J = 2.5, 10 Hz, 0.5 x CH₂); 3.17-3.31 (3H, m) and 3.41-3.52 (2H, m, -CH-oxadiazole, -CH-bridgehead, 0.5 x CH₂ and CH₂); 4.49-4.55 (1H, m, -CH-OH).

### EXAMPLE 3

### Exo-3-[5-(3-Amino-1,2,4-oxadiazol)yl]-1-azabicyclo[2.2.1]heptan-5-ol

### a) 3-[5-(3-Amino-1,2,4-oxadiazol)yl]-5,5-dimethoxy-1-azabicyclo[2.2.1]heptane.

Sodium (0.75 g, 32.6 mmol) was cut into small pieces and added to a stirred mixture of hydroxyguanidine sulphate (2.47 g, 9.30 mmol) in methanol (60 ml, predried over molecular sieves). After 0.25 h a solution of 3-carbomethoxy-5,5-dimethoxy-1-azabicyclo[2.2.1]heptane (1 g, 4.65 mmol) in methanol (5 ml) was added and heated at 80°C for 2 h.
The reaction mixture was decanted from the molecular sieves, the solvent removed under vacuum and the residue taken up into water (10 ml). Extraction into dichloromethane (4 x 100 ml), drying (Na₂SO₄), and removal of solvent under vacuum, was followed by chromatography through alumina (Grade II/III) using dichloromethane/methanol (98:2), as eluant, to afford the title amino oxadiazole (0.26 g); m/e 240 (M⁺)⁺, 225 (⁺M-CH₃); δ (360 MHz, CDCl₃) 2.41 (1H, dd, J = 13 Hz and 3.2 Hz, CH of CH₂); 2.73 (1H, dd, J = 10 Hz and 3.3 Hz, CH of CH₂); 2.91 - 3.19 (5H, m, 2 x CH₂ and CH); 3.21 (3H, s, OMe); 3.26 (3H, s, OMe); 3.35 - 3.68 (1H, m, CH-oxadiazole); 4.36 (2H, brs, NH₂).

### b) 3-[5-(3-Amino-1,2,4-oxadiazol)yl]-1-azabicyclo[2.2.1]heptan-5-one.

A solution of 3-[5-(3-amino-1,2,4-oxadiazol)yl-5,5-dimethoxy-1-azabicyclo[2.2.1]heptane (0.26 g, 1.08 mmol) in perchloric acid (4 ml) was stirred at 85°C for 24 h. The mixture was diluted with water (10 ml), neutralised with sodium carbonate and extracted with dichloromethane (4 x 100 ml). The combined extracts were dried (Na₂SO₄), the solvent evaporated, and the residue washed with cold dichloromethane (50ml) to give the title ketone (0.14 g); δ (360 MHz, d₆-DMSO) 2.72 - 3.33 (7H, m, 3 x CH₂ and CH); 3.40 (1H, dd, J = 8 Hz and 5 Hz, CH-oxadiazole); 6.27 (2H, brs, NH₂);

### c) Exo-3-[5-(3-Amino-1,2,4-oxadiazol)yl]-1-azabicyclo[2.2.1]heptan-5-ol

Sodium borohydride (36 mg, 0.94 mmol) was added to a solution of 3-[5-(3-amino-1,2,4-oxadiazol)yl-1-azabicyclo[2.2.1]heptan-5-one (0.14 g, 0.72 mmol) in ethanol (40 ml), at 0°C. The mixture was stirred at 0°C for 0.5 h and at room temperature for 0.5 h. Excess borohydride was destroyed by addition of 2N hydrochloric acid and the solvent removed under reduced pressure. The residue was taken up into water (10 ml), basified to pH 10 with potassium carbonate, and extracted with dichloromethane (4 x 100ml). Drying (Na₂SO₄) and removal of solvents under vacuum, were followed by chromatography of the residue through alumina (Grade II/III) using dichloromethane/methanol (90:10) as eluant to give exo- 3-[5-(3-amino-1,2,4-oxadiazol)yl]-1-azabicyclo[2.2.1]-heptan-5-ol (20 mg), m.p. 220 - 221.5°C (ethanol); [Found: C, 49.39; H, 6.36; N, 27.64. C₈H₁₂N₄O₂. 0.15 EtOH requires C, 49.08; H, 6.40; N, 27.58%); m/e 196 (M⁺); δ(360 MHz D₂O) 2.15 (1H, d, J = 11 Hz, CH of CH₂); 2.72 (2H, brs, CH₂-N); 3.03 (1H, d, J = 4.4 Hz,CH-bridgehead); 3.04 - 3.21 (3H, m, 2 x CH₂); 3.65 - 3.72 (1H, m, CH-oxadiazole); 4.50 - 4.55 (1H, m, CH-OH).

### EXAMPLE 4

### Exo-3-[5-(3-methyl-1,2,4-oxadiazoi)yl]-5-fluoro-1-azabicyclo[2.2.1]heptane Hydrochloride

Diethylaminosulphur trifluroride (0.21 g, 1.30 mmol) was added dropwise to a solution of exo-3-[5-(3-methyl-1,2,4-oxadiazol)yl]-1-azabicyclo[2.2.1]heptan-5-ol (0.25 g, 1.28 mmol) in dichloromethane (30 ml), at -78°C, and stirred at this temperature for 1 h before allowing to warm to room temperature and stir for 1.5 h. Water (20 ml) was added and basified with potassium carbonate. Extraction into dichloromethane (3 x 100 ml), drying (Na₂SO₄) and removal of solvents under vacuum was followed by chromatography through silica-gel, using dichloromethane/methanol (90:10) as eluant, to give a crude product. Further chromatography through alumina using dichloromethane/methanol (99:1) as eluant gave the title fluoride (50 mg). The hydrochloride salt was prepared, m.p. 197 - 200°C (isopropylalcohol/ether); [Found: C, 45.98; H, 5.60; N, 17.58. C₉H₁₂N₃FO. HC1.O.1H₂O requires C, 45.90; H, 5.61; N, 17.58%]; δ (360 MHz, CDC1₃) 2.36 (3H, s, CH₃); 2.50 - 3.10 (8H, m, 3 x CH₂ and 2 x CH); 4.67 (1H, d, J_{H-F} = 56 Hz, CH-F).

### EXAMPLE 5

### Exo-5-[5-(3-Methyl-1,2,4-oxadiazol)yl]-3-methoxymethyl-1-azabicyclo[2.2.1]heptane Sesquioxalate

### a) 3-Hydroxymethyl-5,5-dimethoxy-1-azabicyclo-[2.2.1]heptane

A solution of 3-carbomethoxy-5,5-dimethoxy-1-azabicyclo[2.2.1]heptane (4.19 g, 19.5 mmol) in anhydrous THF (40 ml) was added dropwise, with stirring, to a solution of lithium aluminium hydride (19.5 ml of 1M solution in THF, 19.5 mmol), in THF (50 ml), at 5°C. Stirring at 5°C for 1 h was followed by stirring at room temperature for 1 h. Water (3 ml) and 2N sodium hydroxide (1 ml) were added and the reaction mixture filtered through hyflo filter aid. The filter pad was washed with dichloromethane (200 ml), the combined organics dried (Na₂SO₄), and evaporated to give 3-hydroxymethyl-5,5-dimethoxy-1-azabicyclo[2.2.1]heptane, (2,83 g) m.p. 151 - 153°C (ethylacetate); [Found: C, 57.54, H, 9.04; N, 7.30. C₉H₁₇NO₃ requires C, 57.73; H, 9.15; N, 7.48%]; δ(360 MHz, CDCl₃) 2.20 - 2.27 (1H, m, CH of CH₂); 2.38 (1H, dd, J = 9 Hz and 2.4 Hz, CH of CH₂); 2.39 - 2.45 (1H, m, CH of CH₂); 2.48 (1H, dd, J = 13 Hz and 3.2 Hz, CH of CH₂); 2.78 (1H, d, J = 4.0 Hz, CH-bridgehead); 2.87 (1H, dd, J = 13 Hz and 1.7 Hz, CH of CH₂); 2.95 (1H, dd, J = 9.6 Hz and 2.3 Hz, CH of CH₂); 3.02 - 3.09 (1H, m, CH); 3.24 (3H, s, OMe); 3.26 (3H, s, OMe); 3.65 -3.82 (2H, m, CH₂-OH).

### b) 3-Methoxymethyl-1-azabicyclo[2.2.1]heptan-5-one

Diethylaminosulphurtrifluoride (0.69 g, 4.3 mmol) was added dropwise to a stirred solution of 3-hydroxymethyl-5,5-dimethoxy-1-azabicyclo[2.2.1]heptane (0.80 g, 4.3 mmol) in dichloromethane (40 ml), at -78°C. After 1 h the reaction mixture was warmed to room temperature and stirred for 16 h. Water (10 ml) and dichloromethane (100 ml) were added and the aqueous basified with potassium carbonate. Extraction into dichloromethane (3 x 100 ml), drying (Na₂SO₄) and evaporation of solvent were followed by chromatography of the residue through silica-gel using dichloromethane/methanol (93:7) as eluant to give the title ketone (0.23 g) as an orange oil; δ(360 MHz, CDCl₃) 2.38 - 2.46 (1H, m, CH of CH₂); 2.64 - 3.02 (6H, m, 2 x CH₂, CH of CH₂ and CH-bridgehead); 3.30 (3H, s, OMe); 3.26 - 3.66 (3H, m, CH₂-OMe and CH).

### c) 5-(1,3-Dithian-2-ylidene)-3-methoxymethyl-1-azabicyclo[2.2.1]heptane

n-Butylithium (2.03 ml of 1.6M solution in hexane, 3.25 mmol) was added to a solution of 2-trimethylsilyl-1,3-propanedithiane (0.68 g, 3.54 mmol) in anhydrous THF (50 ml), at -35°C, and the solution stirred for 2 h. A solution of 3-methoxymethyl-1-azabicyclo[2.2.1]heptan-5-one (0.42 g 2.71 mmol) in THF (5 ml) was added and the reaction mixture warmed to room temperature and stirred for 1 h. Water (6 ml) was added followed by dichloromethane (100 ml) and stirred for 0.1 h. Chromatography of the residue remaining after extraction into dichloromethane (3 x 100 ml), drying (Na₂SO₄) and evaporation of solvent, through silica gel, using dichloromethane/methanol (91:9) as eluant, gave 5-(1,3-dithian-2-ylidene)-3-methoxymethyl-1-azabicyclo[2.2.1]heptane (0.53 g); m/e 157 (M⁺);δ(360 MHz, CDCl₃) 1.96 - 2.20 (4H, m, 2 x CH₂); 2.30 - 3.20 (10H, m, 4 x CH₂ and 2 x CH); 3.32 (3H, s, OMe); 3.40 - 3.54 (2H, m, CH₂-OMe).

### d) 5-Carbomethoxy-3-methoxymethyl-1-azabicyclo[2.2.1]heptane

The preceeding dithioketene acetal (0.53 g, 2.06 mmol) was dissolved in methanol (saturated with hydrogen chloride) (45 ml) and stirred at 55°C for 24 h. The solvent was removed under vacuum, the residue taken up into water (6 ml) and basified with potassium carbonate. The aqueous was extracted into dichloromethane (4 x 70 ml), dried (Na₂SO₄) and the solvents removed under vacuum. The resultant residue was purified by chromatography through alumina (Grade II/III) using dichloromethane/methanol (99:1) as eluant to give the title ester (0.26 g) as an orange oil, m/e 199 (M⁺); δ(360 MHz, CDCl₃) 1.92 - 1.98 (1H, m, CH); 2.29 - 2.86 (5H, m, 2 x CH₂ and CH); 2.87 (1H, d, J = 4 Hz, CH-bridgehead); 2.95 - 3.08 (2H, m, CH₂); 3.35 (3H, s, OMe); 3.29-3.42 (2H, m, CH₂-OMe); 3.68 (3H, s, CO₂Me).

### e) Exo-5-[5-(3-methyl-1,2,4-oxadiazol)yl]-3-methoxy methyl-1-azabicyclo[2.2.1]heptane Sesquioxalate

Sodium hydride (78.4 mg of an 80% dispersion in oil, 2.61 mmol) was added to a stirred solution of methylamide oxime (0.22 g, 2.90 mmol) in anhydrous THF (70 ml) in the presence of molecular sieves (1 g). The reaction mixture was stirred at 75°C for 0.25 h before adding a solution of 5-carbomethoxy-3-methoxymethyl-1-azabicyclo[2.2.1]heptane (0.26 g, 1.31 mmol) in THF (5 ml) and refluxing for 1.5 h. The reaction mixture was cooled to room temperature and water (15 ml) and dichloromethane (100 ml) added. The aqueous was extracted with dichloromethane (4 x 100 ml), the combined extracts dried (Na₂SO₄), and evaporated, and the residue chromatographed through alumina, using dichloromethane/methanol (99:1) as eluant to give the title oxadiazole. The compound was further purified as the sesquioxalate salt, m.p. 122.5 - 124.5°C (dichloromethane/ether); [Found: C, 46.65; H, 5.67; N, 11.14. C₁₁H₁₇N₃O₂.1.6(CO₂H)₂ requires C, 46.41; H, 5.56; N, 11.44%]; δ(360 MHz, D₂O) 2.38 (3H, s, Me); 2.75 - 3.10 (2H, m, CH₂); 3.20 -3.30 (2H, m, 2 x CH); 3.40 (3H, s, OMe); 3.53 - 3.91 (6H, m, 3 x CH₂); 3.90 - 3.96 )1H, m, CH-oxadiazole).

### EXAMPLE 6

### exo-5-[5-(3-methyl-1,2,4-oxadiazol)yl]-3-methyl-1-azabicyclo[2.2.1]heptane Hydrochloride

### a) 3-Methyl-5,5-dimethoxy-1-azabicyclo[2.2.1]heptane

n Butyllithium (2.21ml of a 2.5M solution in hexane, 5.53 mmol) was added to a solution of 3-hydroxymethyl-5,5-dimethoxy-1-azabicyclo[2.2.1]heptane (0.94 g, 5.03 mmol) in THF (50 ml), at 0°C, and stirred for 1 h. Tetramethylphosphoramidic chloride (1.53 ml, 10.05 mmol) was added to the reaction mixture and stirred at room temperature for 1.5 h. Removal of the solvent under vacuum was followed by chromatography through alumina using dichloromethane/methanol (99:1) as eluant to give the desired 3-tetramethylphosphoramidate (1.4 g), δ(360 MHz, CDCl₃) 2.25 - 2.73 (6H, m, 2 x CH₂ and 2 x CH); 2.62 (6H, s, NMe); 2.66 (6H, s, NMe); 2.89 - 3.00 (1H, m, 0 5 x CH₂); 3.18 (6H, s, OMe); 3.20 - 3.27 (1H, m, 0.5 x CH₂); 4.05 - 4.30 (2H, m, CH₂-O).

Lithium metal (0.31 g, 43.6 mmol) was added to freshly distilled ethylamine (50 ml) and stirred at 0°C until the metal had dissolved. A solution of t-butylalcohol (0.97 g, 13.1 mmol) and the preceding phosphoramidate (1.4 g, 4.36 mmol), in anhydrous THF (10 ml) was added to the reaction mixture at such a rate so as to maintain the blue colouration. After stirring for 0.5 h the reaction was quenched by cautious addition of water (50 ml), extracted into dichloromethane (4 x 100 ml), dried (Na₂SO₄) and the solvent evaporated. Chromatography through alumina (Grade II/III) eluting with dichloromethane/methanol (97.5:2.5) gave 3-methyl-5,5 dimethoxy-1-azabicyclo[2.2.1]heptane (0.55 g) as a colourless oil; m/e 171 (M⁺); δ(360 MHz, CDCl₃) 1.30 (3H, d, J = 7.3 Hz, MeCH); 1.98 - 2.04 (1H, m, CH); 2.37 (1H, dd, J = 12.5 Hz and 3.3 Hz, 0.5 x CH₂); 2.43 (1H, dd, J = 9.3 Hz and 3.7 Hz, 0.5 x CH₂); 2.49 (1H, d, J = 3.7 Hz, CH-bridgehead); 2.63 - 2.70 (2H, m, CH₂); 2.91 (1H, dd, J = 9.4 Hz and 2 Hz, 0.5 x CH₂); 2.95 (1H, d, J = 11 Hz, 0.5 x CH₂); 3.18 (3H, s, OMe); 3.20 (3H, s, OMe).

### b) 3-Methyl-1-azabicyclo[2.2.1]heptan-5-one

A solution of 3-methyl-5,5-dimethoxy-1-axabicyclo[2.2.1]heptane (0.55 g, 3.22 mmol) in perchloric acid (3 ml) was stirred for 1h at room temperature. Dichloromethane (40 ml) was added followed by water (10 ml) and the aqueous basified with sodium carbonate. The aqueous was extracted with dichloromethane (4 x 40 ml), the combined extracts dried (Na₂SO₄) and the solvent removed under vacuum to give the title ketone (0.35 g), m/e 125 (M⁺) δ(360 MHz, CDCl₃) 1.05 (3H, d, J = 7 Hz, Me-CH); 2.13 - 2.18 (1H, m, CH); 2.60 - 2.70 (2H, m, CH and 0.5 x CH₂); 2.74 (1H, dd, J = 17.5 and 4.3 Hz, 0.5 x CH₂); 2.87 (1H, dd, J = 10.4 and 4.3 Hz, 0.5 x CH₂): 3.03 (1H, dd, J = 10.4 and 2.6 Hz, 0.5 x CH₂); 3.09 (1H, d, J = 17.5 Hz, 0.5 x CH₂) 3.33 - 3.39 (1H, m, 0.5 x CH₂).

### c) 3-Methyl-5-(1,3-dithian-2-ylidene)-1-azabicyclo[2.2.1]heptane

This was prepared by the procedure described for Example 5 part c. Thus a solution of 3-methyl-1-azabicyclo[2.2.1]heptan-5-one (0.35 g, 2.80 mmol) in THF when treated with 2-trimethylsilyl-2-lithio-1,3-propane dithiane gave the title product (0.44 g), after chromatography through alumina, using dichloromethane/methanol (97:3) as eluant; m/e 227 (M⁺); δ(360 MHz, CDCl₃) 0.96 (3H, d, J = 7 Hz, Me-CH) 2.12 - 2.70 (4H, m, 1.5 x CH₂ and CH); 2.50 - 3.50 (10H, m, CH and 4.5 x CH₂).

### d) 3-Methyl-5-carbomethoxy-1-azabicyclo[2.2.1]heptane

This was prepared by the procedure described for Example 5 part d. Thus treating the preceeding dithioketene acetal (0.44 g, 4.41 mmol) with methanol (saturated with hydrogen chloride) (30 ml) gave, after alumina chromatography, eluting with dichloromethane, the title ester (0.14 g) as a pale yellow liquid m/e 169 (M⁺), δ(360 MHz, CDCl₃) 0.93 (3H, d, J = 7 Hz, Me-CH); 1.75 - 1.80 (2H, m, CH and 0.5 x CH₂); 2.48 - 3.06 (7H, m, 2 x CH and 2.5 x CH₂); 3.68 (3H, s, Me).

### e) exo-5-[5-(3-Methyl-1,2,4-oxadiazol)yl]-3-methyl-1-azabicyclo[2.2.1]heptane Hydrochloride

This was prepared by the procedure described for Example 5 part e. Thus 3-methyl-5-carbomethoxy-1-azabicyclo[2.2.1]heptane (0.14 g, 0.83 mmol) gave the title oxadiazole (40 mg) after chromatography through silica-gel using dichloromethane/methanol (93:7) as eluant and upon treatment with ethereal hydrogen chloride, m.p. 175-177°C, (isopropyl alcohol); [Found: C, 51.81; H, 6.99; N, 17.73. C₁₀H₁₅N₃O.HCl.O.15 H₂O requires C, 51.68; H, 7.02; N, 18.09%]; m/e 193 (M⁺ free base); δ(360 MHz, CDCl₃) 1.25 (3H, d, J = 6.7 Hz, Me-CH); 2.41 (3H, s, Me); 2.75 - 2.84 (2H, m, CH and 0.5 x CH₂); 3.24 (1H, bd, J = 3.5 Hz, CH-bridgehead); 3.42 (1H, d, J = 10 Hz, 0.5 x CH₂); 3.46 (1H, d, J = 10 Hz, 0.5 x CH₂); 3.70 - 3.88 (3H, m, CH₂ and 0.5 x CH₂); 4.04 (1H, dd, J = 8.5 and 5.5 Hz, CH-oxadiazole).

### EXAMPLE 7

### exo-3-[5-(3-Methyl-1,2,4-oxadiazol)yl]-5-hydroxy methyl-1-azabicyclo[2.2.1]heptane Hydrochloride

### a) 3-[5-(3-Methyl-1,2,4-oxadiazol)yl]-5-(1,3-Dithian-2-ylidene)-1-azabicyclo[2.2.1]heptane

This was prepared from 3 [5-(3-methyl-1,2,4-oxadiazol)yl]-1-azabicyclo[2.2.1]heptan-5-one (1.21 g, 6.27 mmol) by the procedure described for Example 5 part c. The product (1.37 g) was obtained as an orange oil, m/e 295 (M⁺); δ(360 MHz, CDCl₃) 2.13 - 2.31 (2H, m, CH₂); 2.38 (3H, s, Me); 2.70 -3.50 (12H, m, 5 x CH₂ and 2 x CH).

### b) 3-[5-(3-Methyl-1,2,4-oxadiazol)yl]-5-carbomethoxy-1-azabicyclo[2.2.1]heptane

This was prepared from the preceding dithioketene acetal (1.37 g, 4.84 mmol) by the procedure described for Example 5 part d. The product (0.8 g) was obtained as a yellow oil; m/e 237 (M⁺); δ(360 MHz, CDCl₃) 2.37 (3H, s, Me); 2.70 - 3.40 (9H, m, 3 x CH₂ and 3 x CH); 3.70 (3H, s, CO₂Me).

### c) exo-3-[5-(3-Methyl-1,2,4-oxadiazol)yl]-5-hydroxymethyl-1-azabicyclo[2.2.1]heptane Hydrochloride

A solution of 3-[5-(3-methyl-1,2,4-oxadiazol)yl]-5-carboxymethoxy-1-azabicyclo[2.2.1]heptane (0.80 g, 3.38 mmol) in anhydrous THF (10 ml) was added dropwise to a stirred solution of diisobutylaluminium hydride (8.44 ml of a 1.0M solution in toluene 8.44 mmol), in THF (50 ml), at -70°C. Stirring at -70°C for 0.25 h was followed by warming to room temperature and stirring for 0.5 h. Aqueous workup, extraction into dichloromethane (5 x 50 ml), drying (Na₂SO₄) and removal of the solvent under vacuum gave the crude product which was purified by chromatography through alumina using dichloromethane/methanol (97:3) as eluant. The product (0.52 g) was obtained on a colourless oil. The hydrochloride salt was prepared, [Found: C, 48.52; H, 5.93; N, 16.83; C₁₀H₁₅N₃O₂.HCl requires C, 48.88; H, 6.11; N, 17.11%]; m/e 209 (M⁺-free base);; δ(360 MHz, CDCl₃); 1.90 (1H, brs, OH). 2.37 (3H, s, Me); 2.38 - 3.20 (9H, m. 3 x CH₂ and 3 x 0.5); 3.50 - 3.69 (2H, m, CH₂-OH).

### EXAMPLE 8

### exo-3-[5-(3-Methyl-1,2,4-oxadiazol)yl]-4-methyl-1-azabicyclo[2.2.1]heptane. Hydrochloride and endo-3-[5-(3-methyl-1,2,4-oxadiazol)yl-4-methyl-1-azabicyclo[2.2.1]heptane Hydrochloride

### a) 4-Methyl-1-azabicyclo[2.2.1]heptan-3-one

A solution of 1-carbomethoxymethyl-3-methyl-3-carbomethoxypyrrolidine (14g, 65mmol) in toluene (150ml) was added over a 0.75h period to a rapidly stirred solution of potassium-t-butoxide (24g, 21mmol) in toluene (750ml), at 140°C for 4h, cooled to room temperature and concentrated hydrochloric acid (250ml) added. The aqueous was separated and the organic extracted with 3 further portions of hydrochloric acid (3 x 150ml). The combined aqueous was heated at 110°C for 8h, the water removed to half the volume, basified with potassium carbonate, and extracted with dichloromethane (4 x 250ml). The combined extracts were dried (Na₂SO₄), evaporated, and the residue chromatographed through alumina, using dichloromethane/methanol (98:2) as eluant to give the title ketone (4g) as a colourless oil. The product was characterised as the hydrochloride salt, m.p. 243-245°C (isopropylalcohol), [Found, C, 51.87; H, 7.24; N, 8.68. C₇H₁₁NO.HCl requires C, 52.02; H, 7.48; N, 8.67%); m/e 126 (M+H)⁺; δ (360MHz, CDCl₃) 1.35 (3H, s, Me); 1.98-2.06 (1H, m, CH of CH₂); 2.29-2.38 (1H, m, CH of CH₂); 3.18-3.62 (3H, m, CH₂ and CH of CH₂); 3.79-3.92 (2H, m, CH₂); 4.06 (1H, dd, J = 2.7 and 17Hz, CH of CH₂).

### b) 3-(1,3-Dithian-2-ylidene)-4-methyl-1-azabicyclo[2.2.1]heptane

n-Butyllithium (20ml of a 1.6M solution in hexane, 32.2mmol) was added to a stirred solution of 2-trimethylsilyl-1,3-dithiane (4.69g, 24.3mmol) in anhydrous THF (150ml), at -50°C. After 2h, a solution of 4-methyl-1-azabicyclo[2.2.1]heptan-3-one (2.54g, 20mmol) in THF (10ml) was added and the reaction mixture warmed to room temperature. Aqueous work up and extraction into dichloromethane gave the crude product which was chromatographed through silica gel using dichloromethane/methanol (93:7) as eluant to give the title compound (1.06g) as a low melting solid, m.p. 48-49°C, m/e 227 (M⁺); δ (360MHz, CDCl₃) 1.64 (3H, s, Me); 1.64-1.91 (2H, m, CH₂); 2.08-2.15 (2H, m, CH₂); 2.38 (1H, dd, J = 3.45 and 9.2 Hz, CH of CH₂); 2.58-3.20 (7H, m, 3 of CH₂ and CH of CH₂); 3.24 (1H, dd, J = 3.5 and 16Hz, CH of CH₂); 3.57 (1H, d, J = 16Hz, CH of CH₂).

### c) 3-Carbomethoxy-4-methyl-1-azabicyclo[2.2.1]heptane

The preceding dithioketeneacetal (1.7g, 7.5mmol) was dissolved in methanol (saturated with hydrogenchloride, 100ml) and stirred at 65°C for 4h. The solvent was removed under vacuum, water (20ml) added and basified to pH10 with potassium carbonate. The aqueous was extracted into dichloromethane (4 x 150ml), dried (Na₂SO₄) and the residue remaining after removal of solvents under vacuum chromatographed through alumina, using dichloromethane/methanol (99:1) as eluant, to give the title ester (0.82g) as a pale yellow oil; δ (360MHz, CDCl₃) 1.22 (3H, s, Me); 1.23-1.27 (1H, m, CH of CH₂); 1.43-1.52 (1H, m, CH of CH₂); 2.12-2.15 (1H, m, CH of CH₂); 2.23-2.26 (1H, m, CH of CH₂); 2.72-2.82 (2H, m, CH₂); 2.93-3.07 (2H, m, CH₂); 3.16-3.21 (1H, m, CH of CH₂); 3.68 (3H, s, CO₂Me).

### d) exo-3-[5-(3-Methyl-1,2,4-oxadiazol)yl]-4-methyl-1-azabicyclo[2.2.1]heptane. Hydrochloride and endo-3-[5-(3-methyl-1,2,4-oxadiazol)yl]-4-methyl-1-azabicyclo[2.2.1]heptane. Hydrochloride

Sodium hydride (0.36g of an 80% dispersion in oil, 12.1mmol) was added to a stirred solution of methylamide oxime in THF (50ml), in the presence of molecular sieves (4A) and the reaction mixture heated at 70°C for 0.25h. A solution of 3-carbomethoxy-4-methyl-1-azabicyclo[2.2.1]heptane (0.82g, 4.85mmol) in THF (15ml) was then added and heated at 70°C for 1.5h. Water (15ml was added followed by dichloromethane (150ml) and the aqueous separated and extracted with dichloromethane (4 x 75ml). The combined extracts were dried (Na₂SO₄) and evaporated, and the residue chromatographed through alumina using dichloromethane/methanol (100-99:1) as eluant to give the title compounds. The mixture of products was further chromatographed through silica-gel using dichloromethane/methanol (95:5) as eluant to give 2 separated components.

The less polar compound was identified as the exo isomer (0.29g) and was further purified as the hydrochloride salt, m.p. 226-227°C (isopropylalcohol); (Found: C, 52.15; H, 6.89; N, 18.08. C₁₀H₁₅N₃O.HCl requires C, 52.29; H, 7.02; N, 18.29%); δ (360MHz, D₂O) 1.13 (3H, s, Me); 2.09-2.18 (2H, m, CH₂); 2.42 (3H, s, Me); 3.23 (1H, d, J = 10Hz, CH of CH₂); 3.45-3.52 (2H, m, CH₂); 3.62-3.70 (1H, m, CH of CH₂); 3.75-3.79 (1H, m, CH of CH₂); 3.82-3.89 (1H, m, CH of CH₂); 3.91-3.97 (1H, m, CH-oxadiazole).

The more polar compound was identified as the second title compound and was characterised as the hydrochloride salt, m.p. 196-197°C (isopropyl alcohol); (Found: C, 51.51: H, 7.18; N, 17.96. C₁₀H₁₅N₃O.HCl.0.2H₂O requires C, 51.48; H, 7.09; N, 18.01%); δ (360MHz, D₂O) 1.50 (3H, s, Me); 1.67-1.76 (1H, m, CH of CH₂); 1.86-1.95 (1H, m, CH of CH₂); 2.43 (3H, s, Me); 3.35 (1H, dd, J = 2.5 and 9.2Hz, CH of CH₂); 3.45-3.53 (2H, m, CH₂); 3.62-3.67 (1H, m, CH of CH₂); 3.80-3.84 (1H, m, CH-oxadiazole); 3.91-4.06 (2H, m, CH₂).

### EXAMPLE 9

### exo-3-[5-(3-Dimethylamino-1,2,4-oxadiazol)yl]-1-azabicyclo[2.2.1]heptan-5-ol

### a) exo-3-[5-(3-Dimethylamino-1,2,4-oxadiazol)yl]-5,5-dimethoxy-1-azabicyclo[2.2.1]heptane

exo-3-[5-(3-Dimethylamino-1,2,4-oxadiazol)yl]-5,5-dimethoxy-1-azabicyclo[2.2.1]heptane was prepared from 3-carbomethoxy-5,5-dimethoxy-1-azabicyclo[2.2.1]heptane (2.73g, 13mmol) and dimethyl hydroxy guanidine hydrochloride (4.35g, 31mmol) by the procedure described for Example 2 part d. The crude product was chromatographed through silica gel, eluting with dichloromethane/methanol (95:5) to give the title dimethylamino oxadiazole as a low melting solid (2.94g), δ (250MHz, CDCl₃) 2.40 (1H, dd, J = 3 and 18Hz, CH of CH₂); 2.75 (1H, dd, J = 3 and 10Hz, CH of CH₂); 2.93 (1H, d, J = 18Hz, CH of CH₂); 2.96 (1H, s, bridgehead-H); 2.97 (1H, d, J = 13Hz, CH of CH₂); 2.99 (6H, s, NMe); 3.04-3.15 (2H, m, CH₂); 3.21 (3H, s, OMe); 3.26 (3H, s, OMe); 3.32-3.37 (1H, m, CH-oxadiazole).

### b) exo-3-[5-(3-Dimethylamino-1,2,4-oxadiazol)yl]-1-azabicyclo[2.2.1]heptan-5-one

Deketalisation of the preceding ketal (0.5g, 1.87mmol) was achieved using perchloric acid 17ml) using the procedure described for Example 2 part e. The crude product was chromatographed through silica-gel using dichloromethane/methanol (95:5) as eluant to give the title ketone (0.37g); δ (250MHz, CDCl₃) 2.88 (1H, dd, J = 4.1 and 17.9Hz, CH of CH₂); 3.00 (6H, s, NMe); 3.09-3 46 (7H, m, 2 of CH₂, CH of CH₂ CH-oxadiazole, and CH-bridgehead).

### c) exo-3-[5-(3-Dimethylamino-1,2,4-oxadiazol)yl]-1-azabicyclo[2.2.1]heptan-5-ol

Sodium borohydride (0.035g, 0.92mmol) was added to a solution of the preceding ketone (0.11g, 0.046mmol) in ethanol (10ml), at 0°C, and stirred for 0.20h. The reaction mixture was warmed to room temperature, stirred for 0.1h, sodium methoxide (0.2g, 3.7mmol) added, and the solution stirred for 1.5h. The solvent was removed under vacuum, water (20ml) added and extracted with ethyl acetate (5 x 50ml). The combined extracts were dried (Na₂SO₄) and evaporated to give the crude product which was recrystallised from ethyl acetate, to give the title compound (55mg) as a white crystalline solid, m.p. 149-150°C; δ (360MHz, CDCl₃) 1.88 (1H, brs, OH); 2.12-2.17 (1H, m, CH of CH₂); 2.63 (1H, d, J = 10.3Hz, CH of CH₂); 2.81 (1H, dd, J = 3.5 and 10.3Hz, CH of CH₂); 2.91 (1H, d, J = 4.1Hz, CH-bridgehead); 3.00 (6H, s, NMe); 3.07-3.25 (3H, m, CH₂ and CH of CH₂); 3.74 (1H, dd, J = 5.3 and 8.3Hz, CH-oxadiazole); 4.47-4.52 (1H, m, CH-OH).

### EXAMPLE 10

### exo-3-[5-(3-Dimethylamino-1,2,4-oxadiazol)yl-5-methyl-1-azabicyclo[2.2.1]heptan-5-ol

Methylmagnesium bromide (0.32ml of a 3M solution in ether, 0.96mmol) was added to a stirred solution of exo-3-[5-(3-dimethylamino-1,2,4-oxadiazol)yl]-1-azabicyclo[2.2.1]heptan-5-one (0.175g, 0.79mmol) in ether (3ml), at 0°C. The reaction mixture was warmed to room temperature stirred for 2h, water (20ml) then added and extracted with dichloromethane (4 x 25ml). The combined extracts were dried (Na₂SO₄) and evaporated to give the title alcohol (0.10g) m.p. 143-147°C; δ (250MHz, CDCl₃) 1.45 (3H, s, Me); 1.70 (1H, br s, OH); 2.43 (1H, dd, J = 3 and 12.7Hz, CH of CH₂); 2.67 (1H, br s, CH-bridgehead); 2.73-2.81 (3H, m, CH₂ and CH of CH₂); 2.99 (6H, s, NMe); 3.04-3.30 (2H, m, CH₂); 3.87 (1H, dd, J = 4.2 and 12.4Hz, CH-oxadiazole).

### EXAMPLE 11

### Tablet Preparation

Tablets containing 1.0, 2.0, 25.0, 26.0, 50.0 and 100.0 mg, respectively, of the following compounds are prepared as illustrated below:
exo-3-[5-(3-Methyl-1,2,4-oxadiazol)yl]-1-azabicyclo[2.2.1]heptan-5-ol.
exo-3-[5-(3-Methyl-1,2,4-oxadiazol)yl]-5-fluoro-1-azabicyclo[2.2.1]heptane. Hydrochloride.
exo-5-[5-(3-Methyl-1,2,4-oxadiazol)yl]-3-methyl-1-azabicyclo[2.2.1]heptane. Hydrochloride.
exo-3-[5-(3-Methyl-1,2,4-oxadiazol)yl]-4-methyl-1-azabicyclo[2.2.1]heptane. Hydrochloride.
exo-3-[5-(3-Dimethylamino-1,2,4-oxadiazol)yl]-1-azabicycio[2.2.1]heptan-5-ol.

### TABLE FOR DOSES CONTAINING FROM 1-25 MG OF THE ACTIVE COMPOUND

| | Amount-mg | | |
|---|---|---|---|
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

### TABLE FOR DOSES CONTAINING FROM 26-100 MG OF THE ACTIVE COMPOUND

| | Amount-mg | | |
|---|---|---|---|
| Active Compound | 26.0 | 50.0 | 100.0 |
| Microcrystalline Cellulose | 52.0 | 100.0 | 200.0 |
| Modified food corn starch | 2.21 | 4.25 | 8.5 |
| Magnesium stearate | 0.39 | 0.75 | 1.5 |

All of the active compound, lactose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.0 mg, 25.0 mg, 26.00 mg, 50.0 mg and 100.0 mg of active ingredient per tablet.

## Claims (Claims for the following Contracting State(s): AT, BE, CH, DE, FR, GB, IT, LI, LU, NL, SE)

1. An oxadiazole represented by structural formula (I): 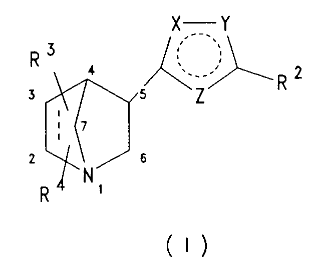 or a salt or prodrug thereof; wherein
one of X, Y or Z is an oxygen atom and the other two are nitrogen atoms, and the dotted circle represents aromaticity (two double bonds);
R² may be hydrogen, halogen, -CF₃, -OR⁷, -NR⁷R⁸, -NHOR⁷, -NHNH₂, -CN, -CO₂R⁷, -CONR⁷R⁸, C₂₋₅ alkenyl, C₂₋₅ alkynyl, C₁₋₂ alkyl, or C₁₋₂ alkyl substituted with -OR⁷, -NR⁷R⁸, -SR⁷, -CO₂R⁷, -CONR⁷R⁸ or halogen;
the broken line represents an optional chemical bond; and
the substituents R³ and R⁴ may be present at the 3-, 4- or 5- position of the azabicyclic ring, including the point of attachment to the oxadiazole ring, and R³ represents halo, C₁₋₄ alkoxy, carboxy, -NR⁷R⁸, C₁₋₄ alkyl, C₁₋₄ alkyl substituted with hydroxy or C₁₋₄ alkoxy, or hydroxy; and R⁴ represents hydrogen, halo, C₁₋₄ alkoxy, hydroxy, carboxy, -NR⁷R⁸, C₁₋₄ alkyl, or C₁₋₄ alkyl substituted with hydroxy or C₁₋₄ alkoxy; or R³ and R⁴ together represent carbonyl; and
R⁷ and R⁸ independently represent hydrogen or C₁₋₂ alkyl.

2. An oxadiazole as claimed in claim 1 represented by formula IIA: 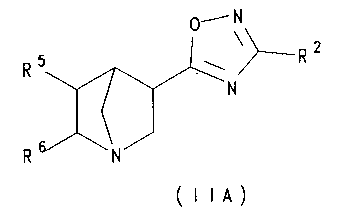 wherein R² is as defined in claim 1, R⁵ represents C₁₋₄ alkyl, halo, -NR⁷R⁸, C₁₋₄ alkoxy, hydroxy(C₁₋₄)alkyl, C₁₋₄ alkoxy(C₁₋₄)alkyl or hydroxy; and R⁶ represents hydrogen, C₁₋₄ alkyl, C₁₋₄ alkoxy or hydroxy; and
R⁷ and R⁸ independently represent hydrogen or C₁₋₂ alkyl.

3. An oxadiazole as claimed in claim 1 represented by formula IIB: 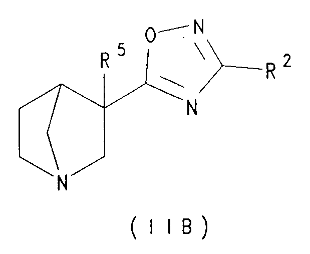 wherein R² is as defined in claim 1 and R⁵ is as defined in claim 2.

4. An oxadiazole as claimed in claim 1 represented by formula IIC: 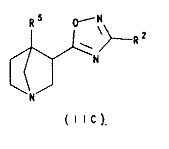 wherein R² is as defined in claim 1 and R⁵ is as defined in claim 2.

5. An oxadiazole as claimed in any of claims 1 to 4 wherein R³ represents halo, C₁₋₄ alkoxy, carboxy, -NR⁷R⁸, C₂₋₄ alkyl, C₁₋₄ alkyl substituted with hydroxy or C₁₋₄ alkoxy, or methyl or hydroxy in the 3-, 4- or 5-position; and R⁴ represents hydrogen, halo, C₁₋₄ alkoxy, hydroxy, carboxy, -NR⁷R⁸, C₁₋₄ alkyl, or C₁₋₄ alkyl substituted with hydroxy or C₁₋₄ alkoxy; or R³ and R⁴ together represent carbonyl.

6. An oxadiazole as claimed in any one of claims 1 to 5 wherein R⁴ is hydrogen or methyl; and R³ is C₁₋₄ alkoxy, halo, -NR⁷R⁸, hydroxy(C₁₋₄)alkyl or C₁₋₄ alkoxymethyl.

7. An oxadiazole as claimed in any of claims 1 to 6 wherein R³ represents methoxy, fluoro, amino, methoxymethyl or hydroxymethyl.

8. An oxadiazole as claimed in any of claims 1 to 7 wherein R⁴ represents halogen.

9. A compound as claimed in claim 1 selected from the following:
3-[5-(3-methyl-1,2,4-oxadiazol)-yl]-1-azabicyclo[2.2.1]heptan-3-ol;
3-[5-(3-methyl-1,2,4-oxadiazol)-yl]-1-azabicyclo[2.2.1]heptan-5-ol;
3-[5-(3-methyl-1,2,4-oxadiazol)-yl]-5-ethyl-1-azabicyclo[2.2.1]heptane;
3-[5-(3-methyl-1,2,4-oxadiazol)-yl]-6-methyl-1-azabicyclo[2.2.1]-heptane;
3-[5-(3-amino-1,2,4-oxadiazol)-yl]-1-azabicyclo[2.2.1]heptan-5-ol;
3-[5-(3-methyl-1,2,4-oxadiazol)-yl]-5-fluoro-1-azabicyclo[2.2.1]heptane;
5-[5-(3-methyl-1,2,4-oxadiazol)-yl]-3-methoxymethyl-1-azabicyclo-[2.2.1]heptane;
5-[5-(3-methyl-1,2,4-oxadiazol)-yl]-3-methyl-1-azabicyclo[2.2.1]heptane;
3-[5-(3-methyl-1,2,4-oxadiazol)-yl]-5-hydroxymethyl-1-azabicyclo-[2.2.1]heptane;
3-[5-(3-dimethylamino-1,2,4-oxadiazol)-yl]-1-azabicyclo[2.2.1]heptan-5-ol;
3-[5-(3-dimethylamino-1,2,4-oxadiazol)-yl]-5-methoxy-1-azabicyclo[2.2.1]heptane;
3-[5-(3-dimethylamino-1,2,4-oxadiazol)-yl]-5-methyl-1-azabicyclo[2.2.1]heptan-5-ol;
3-[5-(3-dimethylamino-1,2,4-oxadiazol)-yl]-5-amino-1-azabicyclo[2.2.1]heptane; and
3-[5-(3-methyl-1,2,4-oxadiazol)-yl]-4-methyl-1-azabicyclo-[2.2.1]heptane;
and salts and prodrugs thereof.

10. A compound as claimed in claim 1 selected from
3-[5-(3-methyl-1,2,4-oxadiazol)-yl]1-azabicyclo-[2.2.1]-heptan-5-ol;
-3-[5-(3-methyl-1,2,4-oxadiazol)-yl]1-azabicyclo-[2.2.1]-heptan-3-ol;
3-[5-(3-methyl-1,2,4-oxadiazol)-yl]-4-methyl-1-azabicyclo-[2.2.1]heptane;
3-[5-(3-methyl-1,2,4-oxadiazol)-yl]-5-ethyl-1-azabicyclo[2.2.1]-heptane;
-3-[5-(3-methyl-1,2,4-oxadiazol)-yl]-6-methyl-1-azabicyclo[2.2.1]heptane;
Exo-3-[5-(3-amino-1,2,4-oxadiazol)-yl]-1-azabicyclo[2.2.1]heptan-5-ol;
3-[5-(3-amino-1,2,4-oxadiazol)-yl]-5,5-dimethoxy-1-azabicyclo[2.2.1]heptane;
3-[5-(3-amino-1,2,4-oxadiazol)-yl]-1-azabicyclo[2.2.1]heptan-5-one;
Exo-3-[5-(3-methyl-1,2,4-oxadiazol)-yl]-5-fluoro-1-azabicyclo[2.2.1]heptane;
Exo-5-[5-(3-methyl-1,2,4-oxadiazol)-yl]-3-methoxymethyl-1-azabicyclo[2.2.1]heptane;
Exo-5-[5-(3-methyl-1,2,4-ozadiazol)-yl]-3-methyl-1-azabicyclo[2.2.1]heptane; and
Exo-3-[5-(3-methyl-1,2,4-oxadiazol)-yl]-5-hydroxymethyl-1-azabicyclo[2.2.1]heptane;
and salts and prodrugs thereof.

11. A compound as claimed in claim 1 selected from
3-[5-(3-methyl-1,2,4-oxadiazol)yl]-1-azabicyclo-[2.2.1]-heptan-5-ol;
Exo-3-[5-(3-methyl-1,2,4-oxadiazol)yl]-1-azabicyclo[2.2.1]-heptan-3-ol;
and salts and prodrugs thereof.

12. 3-[5-(3-Methyl-1,2,4-oxadiazol)yl]-6-methyl-1-azabicyclo[2.2.1]heptane, and salts and prodrugs thereof.

13. A pharmaceutical composition comprising a compound as claimed in any one of the preceding claims in association with a pharmaceutically acceptable carrier.

14. A pharmaceutical composition as claimed in claim 13 further comprising a peripheral cholinergic antagonist.

15. The use of a compound as claimed in any one of claims 1 to 12 for the preparation of a medicament for the treatment of neurological and mental disorders or for the treatment of severe pain.

16. A process for the preparation of a compound as claimed in any one of claims 1 to 12, which process comprises reacting a reactive derivative of a carboxylic acid of formula R^{a}-CO₂H with either a compound of formula IIIA or a compound of formula IIIB or a salt thereof: 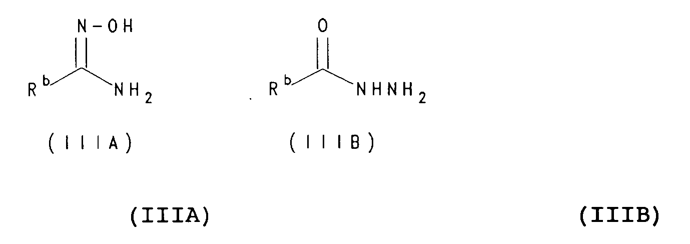 wherein one of R^{a} and R^{b} is a 1-azabicyclo[2.2.1]heptane ring substituted by one or more substituents R³ and R⁴ as defined in claim 1, and the other is a group of low lipophilicity, as defined in claim 1.

17. An intermediate of formula (V) 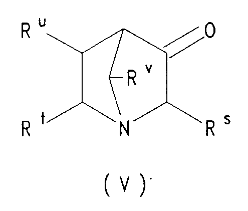 wherein R^{s}, R^{t}, R^{u} and R^{v} represent hydrogen, carboxylic acid ester, or a substituent R³ or R⁴ as defined in claim 1, or a group which is convertible thereto, at least one of R^{s}, R^{t}, R^{u} and R^{v} being other than hydrogen.

18. A process for the preparation of a compound as claimed in claim 17, which process comprises cyclising a compound of formula IV: 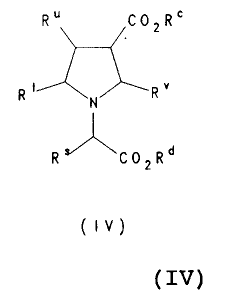 wherein R^{s}, R^{t}, R^{u} and R^{v} are as defined in claim 17, and R^{c} and R^{d} represent hydrocarbon groups.

## Claims (Claims for the following Contracting State(s): ES, GR)

1. A process for the preparation of an oxadiazole represented by structural formula (I): 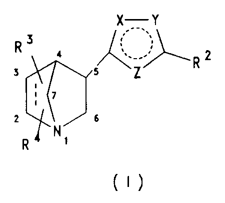 or a salt or prodrug thereof; wherein
one of X, Y or Z is an oxygen atom and the other two are nitrogen atoms, and the dotted circle represents aromaticity (two double bonds);
R² may be hydrogen, halogen, -CF₃, -OR⁷, -NR⁷R⁸, -NHOR⁷, -NHNH₂, -CN, -CO₂R⁷, -CONR⁷R⁸, C₂₋₅ alkenyl, C₂₋₅ alkynyl, C₁₋₂ alkyl, or C₁₋₂ alkyl substituted with -OR⁷, -NR⁷R⁸, -SR⁷, -CO₂R⁷, -CONR⁷R⁸ or halogen;
the broken line represents an optional chemical bond; and
the substituents R³ and R⁴ may be present at the 3-, 4- or 5- position of the azabicylic ring, including the point of attachment to the oxadiazole ring, and R³ represents halo, C₁₋₄ alkoxy, carboxy, -NR⁷R⁸, C₁₋₄ alkyl, C₁₋₄ alkyl substituted with hydroxy or C₁₋₄ alkoxy, or hydroxy; and R⁴ represents hydrogen, halo, C₁₋₄ alkoxy, hydroxy, carboxy, -NR⁷R⁸, C₁₋₄ alkyl, or C₁₋₄ alkyl substituted with hydroxy or C₁₋₄ alkoxy; or R³ and R⁴ together represent carbonyl; and
R⁷ and R⁸ independently represent hydrogen or C₁₋₂ alkyl;
which process comprises reacting a reactive derivative of a carboxylic acid of formula R^{a}-CO₂H with either a compound of formula IIIA or a compound of formula IIIB or a salt thereof: 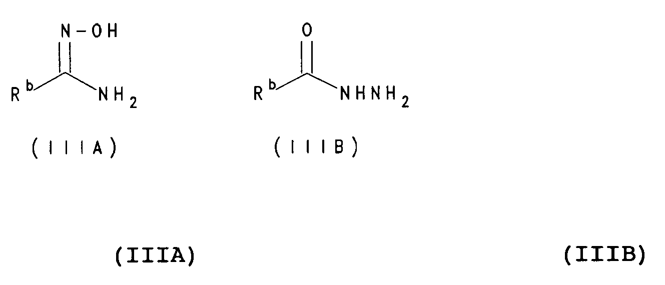 wherein one of R^{a} and R^{b} is a 1-azabicyclo[2.2.1]heptane ring substituted by one or more substituents R³ and R⁴ as defined above, and the other is a group of low lipophilicity, as defined above.

2. A process as claimed in claim 1 wherein the oxadiazole is represented by formula IIA: 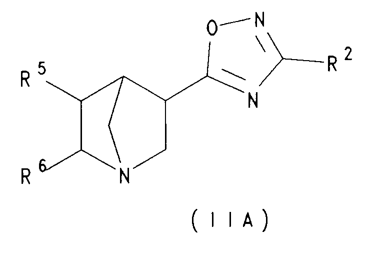 wherein R² is as defined in claim 1, R⁵ represents C₁₋₄ alkyl, halo, -NR⁷R⁸, C₁₋₄ alkoxy, hydroxy(C₁₋₄)alkyl, C₁₋₄ alkoxy(C₁₋₄)alkyl or hydroxy; and R⁶ represents hydrogen, C₁₋₄ alkyl, C₁₋₄ alkoxy or hydroxy; and
R⁷ and R⁸ independently represent hydrogen or C₁₋₂ alkyl.

3. A process as claimed in claim 1 wherein the oxadiazole is represented by formula IIB: 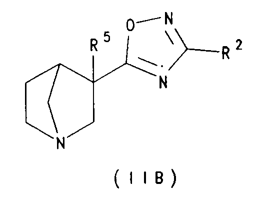 wherein R² is as defined in claim 1 and R⁵ is as defined in claim 2.

4. A process as claimed in claim 1 wherein the oxadiazole is represented by formula IIC: 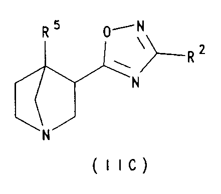 wherein R² is as defined in claim 1 and R⁵ is as defined in claim 2.

5. A process as claimed in any of claims 1 to 4 wherein R³ represents halo, C₁₋₄ alkoxy, carboxy, -NR⁷R⁸, C₂₋₄ alkyl, C₁₋₄ alkyl substituted with hydroxy or C₁₋₄ alkoxy, or methyl or hydroxy in the 3-, 4- or 5-position; and R⁴ represents hydrogen, halo, C₁₋₄ alkoxy, hydroxy, carboxy, -NR⁷R⁸, C₁₋₄ alkyl, or C₁₋₄ alkyl substituted with hydroxy or C₁₋₄ alkoxy; or R³ and R⁴ together represent carbonyl.

6. A process as claimed in any one of claims 1 to 5 wherein R⁴ is hydrogen or methyl; and R³ is C₁₋₄ alkoxy, halo, -NR⁷R⁸, hydroxy (C₁₋₄) alkyl or C₁₋₄ alkoxymethyl.

7. A process as claimed in any of claims 1 to 6 wherein R³ represents methoxy, fluoro, amino, methoxymethyl or hydroxymethyl.

8. A process as claimed in any of claims 1 to 7 wherein R⁴ represents hydrogen.

9. A process as claimed in claim 1 wherein the oxadiazole is selected from:
3-[5-(3-methyl-1,2,4-oxadiazol)-yl]-1-azabicyclo[2.2.1]heptan-3-ol;
3-[5-(3-methyl-1,2,4-oxadiazol)-yl]-1-azabicyclo[2.2.1]heptan-5-ol;
3-[5-(3-methyl-1,2,4-oxadiazol)-yl]-5-ethyl-1-azabicyclo[2.2.1]heptane;
3-[5-(3-methyl-1,2,4-oxadiazol)-yl]-6-methyl-1-azabicyclo[2.2.1]-heptane;
3-[5-(3-amino-1,2,4-oxadiazol)-yl]-1-azabicyclo[2.2.1]heptan-5-ol;
3-[5-(3-methyl-1,2,4-oxadiazol)-yl]-5-fluoro-1-azabicyclo[2.2.1]heptane;
5-[5-(3-methyl-1,2,4-oxadiazol)-yl]-3-methoxymethyl-1-azabicyclo-[2.2.1]heptane;
5-[5-(3-methyl-1,2,4-oxadiazol)-yl]-3-methyl-1-azabicyclo[2.2.1]heptane;
3-[5-(3-methyl-1,2,4-oxadiazol)-yl]-5-hydroxymethyl-1-azabicyclo-[2.2.1]heptane;
3-[5-(3-dimethylamino-1,2,4-oxadiazol)-yl]-1-azabicyclo[2.2.1]heptan-5-ol;
3-[5-(3-dimethylamino-1,2,4-oxadiazol)-yl]-5-methoxy-1-azabicyclo[2.2.1]heptane;
3-[5-(3-dimethylamino-1,2,4-oxadiazol)-yl]-5-methyl-1-azabicyclo[2.2.1]heptan-5-ol;
3-[5-(3-dimethylamino-1,2,4-oxadiazol)-yl]-5-amino-1-azabicyclo[2.2.1]heptane; and
3-[5-(3-methyl-1,2,4-oxadiazol)-yl]-4-methyl-1-azabicyclo-[2.2.1]heptane;
and salts and prodrugs thereof.

10. A process as claimed in claim 1, wherein the oxadiazole is selected from:
3-[5-(3-methyl-1,2,4-oxadiazol)-yl]1-azabicyclo-[2.2.1]-heptan-5-ol;
-3-[5-(3-methyl-1,2,4-oxadiazol)-yl]1-azabicyclo-[2.2.1]-heptan-3-ol;
3-[5-(3-methyl-1,2,4-oxadiazol)-yl]-4-methyl-1-azabicyclo-[2.2.1]heptane;
3-[5-(3-methyl-1,2,4-oxadiazol)-yl]-5-ethyl-1-azabicyclo[2.2.1]-heptane;
-3-[5-(3-methyl-1,2,4-oxadiazol)-yl]-6-methyl-1-azabicyclo[2.2.1]heptane;
Exo-3-[5-(3-amino-1,2,4-oxadiazol)-yl]-1-azabicyclo[2.2.1]heptan-5-ol;
3-[5-(3-amino-1,2,4-oxadiazol)-yl]-5,5-dimethoxy-1-azabicyclo[2.2.1]heptane;
3-[5-(3-amino-1,2,4-oxadiazol)-yl]-1-azabicyclo[2.2.1]heptan-5-one;
Exo-3-[5-(3-methyl-1,2,4-oxadiazol)-yl]-5-fluoro-1-azabicyclo[2.2.1]heptane;
Exo-5-[5-(3-methyl-1,2,4-oxadiazol)-yl]-3-methoxymethyl-1-azabicyclo[2.2.1]heptane;
Exo-5-[5-(3-methyl-1,2,4-ozadiazol)-yl]-3-methyl-1-azabicyclo[2.2.1]heptane; and
Exo-3-[5-(3-methyl-1,2,4-oxadiazol)-yl]-5-hydroxymethyl-1-azabicyclo[2.2.1]heptane;
and salts and prodrugs thereof.

11. A process as claimed in claim 1, wherein the oxadiazole is selected from:
3-[5-(3-methyl-1,2,4-oxadiazol)yl]-1-azabicyclo-[2.2.1]-heptan-5-ol;
Exo-3-[5-(3-methyl-1,2,4-oxadiazol)yl]-1-azabicyclo[2.2.1]-heptan-3-ol;
and salts and prodrugs thereof.

12. A process as claimed in claim 1, wherein the oxadiazole is 3-[5-(3-Methyl-1,2,4-oxadiazol)yl]-6-methyl-1-azabicyclo[2.2.1]heptane, or a salt or prodrug thereof.

13. A process for preparing a pharmaceutical composition comprising preparing an oxadiazole as claimed in any one of the preceding claims and bringing it into association with a pharmaceutically acceptable carrier.

14. A process for the preparation of an intermediate of formula (V) 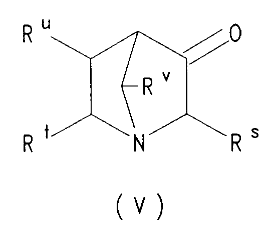 wherein R^{s}, R^{t}, R^{u} and R^{v} represent hydrogen, carboxylic acid ester, or a substituent R³ or R⁴ as defined in claim 1, or a group which is convertible thereto, at least one of R^{s}, R^{t}, R^{u} and R^{v} being other than hydrogen, which process comprises cyclising a compound of formula IV: 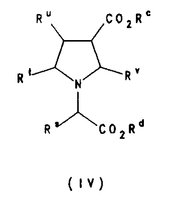 wherein R^{s}, R^{t}, R^{u} and R^{v} are as defined above, and R^{c} and R^{d} represent hydrocarbon groups.

## Patentansprüche (Patentansprüche für folgende(n) Vertragsstaat(en): AT, BE, CH, DE, FR, GB, IT, LI, LU, NL, SE)

1. Oxadiazol, dargestellt durch die Strukturformel (I): 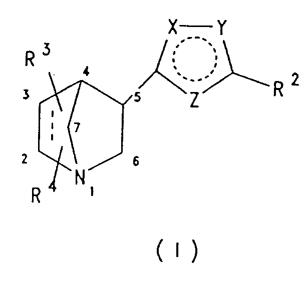 oder ein Salz oder eine Arzneimittelvorstufe davon;
worin
eines von X, Y oder Z für ein Sauerstoffatom steht und die anderen zwei für Stickstoffatome stehen und der gestrichelte Kreis Aromatizität bedeutet (zwei Doppelbindungen);
R² Wasserstoff, Halogen, -CF₃, -OR⁷, -NR⁷R⁸, -NHOR⁷, -NHNH₂, -CN, -CO₂R⁷, -CONR⁷R⁸, C₂₋₅-Alkenyl, C₂₋₅-Alkynyl, C₁₋₂-Alkyl oder C₁₋₂-Alkyl, substituiert mit -OR⁷, NR⁷R⁸, -SR⁷, -CO₂R⁷, -CONR⁷R⁸ oder Halogen darstellen kann.
die durchbrochene Linie eine optionale chemische Bindung bedeutet; und
die Substituenten R³ und R⁴ in Position 3, 4 oder 5 des azabicyclischen Rings, einschließlich des Verknüpfungspunktes mit dem Oxadiazolring, vorhanden sein können;
und R³ für Halogen, C₁₋₄-Alkoxy, Carboxy, -NR⁷R⁸, C₁₋₄-Alkyl, C₁₋₄-Alkyl, substituiert mit Hydroxy oder C₁₋₄-Alkoxy oder Hydroxy steht; und
R⁴ Wasserstoff, Halogen, C₁₋₄-Alkoxy, Hydroxy, Carboxy, -NR⁷R⁸, C₁₋₄-Alkyl oder C₁₋₄-Alkyl, substituiert mit Hydroxy, oder C₁₋₄-Alkoxy bedeutet oder R³ und R⁴ zusammengenommen Carbonyl darstellen; und
R⁷ und R⁸ unabhängig für Wasserstoff oder C₁₋₂-Alkyl stehen.

2. Oxadiazol nach Anspruch 1, dargestellt durch Formel IIA: 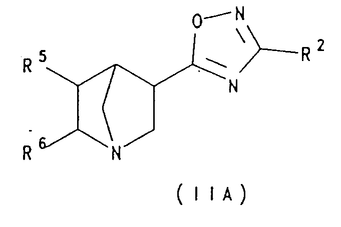 worin R² die in Anspruch 1 gegebene Definition besitzt,
R⁵ für C₁₋₄-Alkyl, Halogen, -NR⁷R⁸, C₁₋₄-Alkoxy, Hydroxy-(C₁₋₄)-alkyl, C₁₋₄-Alkoxy-(C₁₋₄)-alkyl oder Hydroxy steht und R⁶ Wasserstoff, C₁₋₄-Alkyl, C₁₋₄-Alkoxy oder Hydroxy darstellt; und
R⁷ und R⁸ unabhängig für Wasserstoff oder C₁₋₂-Alkyl stehen.

3. Oxadiazol nach Anspruch 1, dargestellt durch Formel IIB: 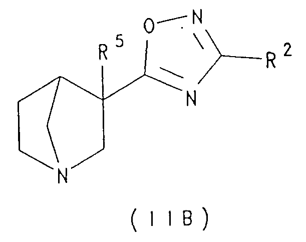 worin R² die in Anspruch 1 gegebene Definition besitzt und R⁵ die in Anspruch 2 gegebene Definition besitzt.

4. Oxadiazol nach Anspruch 1, dargestellt durch Formel IIC: 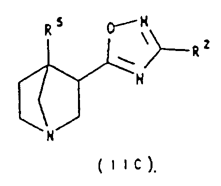 worin R² die in Anspruch 1 gegebene Definition besitzt und R⁵ die in Anspruch 2 gegebene Definition besitzt.

5. Oxadiazol nach einem der Ansprüche 1 bis 4, worin R³ Halogen, C₁₋₄-Alkoxy, Carboxy, -NR⁷R⁸, C₂₋₄-Alkyl, C₁₋₄-Alkyl, substituiert mit Hydroxy, oder C₁₋₄-Alkoxy oder Methyl oder Hydroxy in der Position 3, 4 oder 5 darstellt; und R⁴ für Wasserstoff, Halogen,
C₁₋₄-Alkoxy, Hydroxy, Carboxy, -NR⁷R⁸, C₁₋₄-Alkyl oder C₁₋₄-Alkyl, substituiert mit Hydroxy, oder C₁₋₄-Alkoxy steht; oder R³ und R⁴ zusammen Carbonyl darstellen.

6. Oxadiazol nach einem der Ansprüche 1 bis 5, worin R⁴ Wasserstoff oder Methyl steht und R³ C₁₋₄-Alkoxy, Halogen, -NR⁷R⁸, Hydroxy-(C₁₋₄)-alkyl oder C₁₋₄-Alkoxymethyl bedeutet.

7. Oxadiazol nach einem der Ansprüche 1 bis 6, worin R³ Methoxy, Fluor, Amino, Methoxymethyl oder Hydroxymethyl bedeutet.

8. Oxadiazol nach einem der Ansprüche 1 bis 7, worin R⁴ für Halogen steht.

9. Verbindung nach Anspruch 1, ausgewählt aus folgendem:
3-[5-(3-Methyl-1,2,4-oxadiazol)-yl]-1-azabicyclo[2.2.1]heptan-3-ol;
3-[5-(3-Methyl-1,2,4-oxadiazol)-yl]-1-azabicyclo[2.2.1]heptan-5-ol;
3-[5-(3-Methyl-1,2,4-oxadiazol)-yl]-5-ethyl-1-azabicyclo[2.2.1]heptan;
3-[5-(3-Methyl-1,2,4-oxadiazol)-yl]-6-methyl-1-azabicyclo[2.2.1]heptan;
3-[5-(3-Amino-1,2,4-oxadiazol)-yl]-1-azabicyclo[2.2.1]heptan-5-ol;
3-[5-(3-Methyl-1,2,4-oxadiazol)-yl]-5-fluor-1-azabicyclo[2.2.1]heptan;
5-[5-(3-Methyl-1,2,4-oxadiazol)-yl]-3-methoxymethyl-1-azabicyclo[2.2.1]heptan;
5-[5-(3-Methyl-1,2,4-oxadiazol)-yl]-3-methyl-1-azabicyclo[2.2.1]heptan;
3-[5-(3-Methyl-1,2,4-oxadiazol)-yl]-5-hydroxymethyl-1-azabicyclo[2.2.1]heptan;
3-[5-(3-Dimethylamino-1,2,4-oxadiazol)-yl]-1-azabicyclo-[2,.2.1]heptan-5-ol;
3-[5-(3-Dimethylamino-1,2,4-oxadiazol)-yl]-5-methoxy-1-azabicyclo[2.2.1]heptan;
3-[5-(3-Dimethylamino-1,2,4-oxadiazol)-yl]-5-methyl-1-azabicyclo[2.2.1]heptan-5-ol;
3-[5-(3-Dimethylamino-1,2,4-oxadiazol)-yl]-5-amino-1-azabicyclo[2.2.1]heptan und
3-[5-(3-Methyl-1,2,4-oxadiazol)-yl]-4-methyl-1-azabicyclo[2.2.1]heptan;
und Salze und Arzneimittelvorstufen davon.

10. Verbindung nach Anspruch 1, ausgewählt aus:
3-[5-(3-Methyl-1,2,4-oxadiazol)-yl]-1-azabicyclo[2.2.1]heptan-5-ol;
3-[5-(3-Methyl-1,2,4-oxadiazol)-yl]-1-azabicyclo[2.2.1]heptan-3-ol;
3-[5-(3-Methyl-1,2,4-oxadiazol)-yl]-4-methyl-1-azabicyclo[2.2.1]heptan;
3-[5-(3-Methyl-1,2,4-oxadiazol)-yl]-5-ethyl-1-azabicyclo[2.2.1]heptan;
3-[5-(3-Methyl-1,2,4-oxadiazol)-yl]-6-methyl-1-azabicyclo[2.2.1]heptan;
exo-3-[5-(3-Amino-1,2,4-oxadiazol)-yl]-1-azabicyclo[2.2.1]heptan-5-ol;
3-[5-(3-Amino-1,2,4-oxadiazol)-yl]-5,5-dimethoxy-1-azabicyclo[2.2.1]heptan;
3-[5-(3-Amino-1,2,4-oxadiazol)-yl]-1-azabicyclo[2.2.1]heptan-5-on;
exo-3-[5-(3-Methyl-1,2,4-oxadiazol)-yl]-5-fluoro-1-azabicyclo[2.2.1]heptan;
exo-5-[5-(3-Methyl-1,2,4-oxadiazol)-yl]-3-methoxymethyl-1-azabicyclo[2.2.1]heptan;
exo-5-[5-(3-Methyl-1,2,4-oxadiazol)-yl]-3-methyl-1-azabicyclo[2.2.1]heptan und
exo-3-[5-(3-Methyl-1,2,4-oxadiazol)-yl]-5-hydroxymethyl-1-azabicyclo[2.2.1]heptan
und Salze und Arzneimittelvorstufen davon.

11. Verbindung nach Anspruch 1, ausgewählt aus
3-[5-(3-Methyl-1,2,4-oxadiazol)-yl]-1-azabicyclo[2,2,1]heptan-5-ol;
exo-3-[5-(3-Methyl-1,2,4-oxadiazol)-yl]-1-azabicyclo[2,2,1]heptan-3-ol;
und Salze und Arzneimittelvorstufen davon.

12. 3-[5-(3-Methyl-1,2,4-oxadiazol)-yl]-6-methyl-1-azabicyclo-[2,2,1]heptan und Salze und Arzneimittelvorstufen davon.

13. Pharmazeutisches Präparat, enthaltend eine Verbindung nach einem der vorgenannten Ansprüche, in Verbindung mit einem pharmazeutisch verträglichen Trägerstoff.

14. Pharmazeutisches Präparat nach Anspruch 13, das außerdem einen peripheren cholinergen Antagonisten enthält.

15. Verwendung einer Verbindung nach einem der Ansprüche 1 bis 12 zur Herstellung eines Medikaments zur Behandlung von neurologischen und mentalen Störungen oder zur Behandlung starker Schmerzen.

16. Verfahren zur Herstellung einer Verbindung nach einem der Ansprüche 1 bis 12, wobei das Verfahren umfaßt Umsetzen eines reaktiven Derivats einer Carbonsäure der Formel R^{a}-CO₂H mit entweder einer Verbindung der Formel IIIA oder einer Verbindung der Formel IIIB oder einem Salz davon: 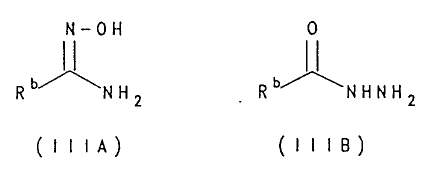 worin eines von R^{a} und R^{b} ein 1-Azabicyclo[2,2,1]-heptan-Ring darstellt, der substituiert ist mit einem oder mehreren Substituenten R³ und R⁴ nach Anspruch 1, und das andere eine Gruppe mit niedriger Lipophilie nach Anspruch 1 ist.

17. Zwischenstufe der Formel (V) 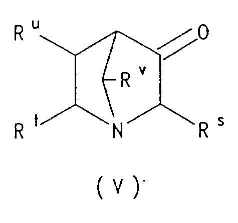 worin R^{s}, R^{t}, R^{u} und R^{v} für Wasserstoff, Carbonsäureester oder einen Substituenten R³ oder R⁴ nach Anspruch 1 oder eine Gruppe, die dazu umwandelbar ist, stehen, wobei mindestens eines von R^{s}, R^{t}, R^{u} und R^{v} etwas anderes als Wasserstoff bedeutet.

18. Verfahren zur Herstellung einer Verbindung nach Anspruch 17, wobei das Verfahren umfaßt Cyclisieren einer Verbindung der Formel IV: 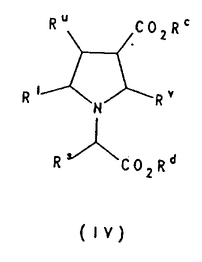 worin R^{s}, R^{t}, R^{u} und R^{v} die in Anspruch 17 gegebenen Definitionen besitzen und R^{c} und R^{d} Kohlenwasserstoffgruppen darstellen.

## Patentansprüche (Patentansprüche für folgende(n) Vertragsstaat(en): ES, GR)

1. Verfahren zur Herstellung eines Oxadiazols, dargestellt durch die Strukturformel (I): 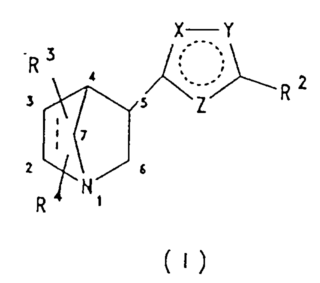 oder ein Salz oder eine Arzneimittelvorstufe davon;
worin
eines von X, Y oder Z für ein Sauerstoffatom steht und die anderen zwei für Stickstoffatome stehen und der gestrichelte Kreis Aromatizität bedeutet (zwei Doppelbindungen);
R² Wasserstoff, Halogen, -CF₃, -OR⁷, -NR⁷R⁸, -NHOR⁷, -NHNH₂, -CN, -CO₂R⁷, -CONR⁷R⁸, C₂₋₅-Alkenyl, C₂₋₅-Alkynyl, C₁₋₂-Alkyl oder C₁₋₂-Alkyl, substituiert mit -OR⁷, NR⁷R⁸, -SR⁷, -CO₂R⁷, -CONR⁷R⁸ oder Halogen darstellen kann.
die durchbrochene Linie eine optionale chemische Bindung bedeutet; und
die Substituenten R³ und R⁴ in Position 3, 4 oder 5 des azabicyclischen Rings, einschließlich des Verknüpfungspunktes mit dem Oxadiazolring, vorhanden sein können;
und R³ für Halogen, C₁₋₄-Alkoxy, Carboxy, -NR⁷R⁸, C₁₋₄-Alkyl, C₁₋₄-Alkyl, substituiert mit Hydroxy oder C₁₋₄-Alkoxy oder Hydroxy steht; und
R⁴ Wasserstoff, Halogen, C₁₋₄-Alkoxy, Hydroxy, Carboxy, -NR⁷R⁸, C₁₋₄-Alkyl oder C₁₋₄-Alkyl, substituiert mit Hydroxy, oder C₁₋₄-Alkoxy bedeutet oder R³ und R⁴ zusammengenommen Carbonyl darstellen; und
R⁷ und R⁸ unabhängig für Wasserstoff oder C₁₋₂-Alkyl stehen, wobei das Verfahren umfaßt Umsetzen eines reaktiven Derivats einer Carbonsäure der Formel R^{a}-CO₂H mit entweder einer Verbindung der Formel IIIA oder einer Verbindung der Formel IIIB oder einem Salz davon: 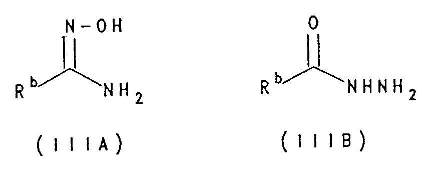 worin eines von R^{a} und R^{b} ein 1-Azabicyclo[2,2,1]-heptan-Ring darstellt, der substituiert ist mit einem oder mehreren Substituenten R³ und R⁴ nach Anspruch 1, und das andere eine Gruppe mit niedriger Lipophilie, wie oben definiert, ist.

2. Verfahren nach Anspruch 1, bei dem das Oxadiazol durch die Formel IIA dargestellt wird, 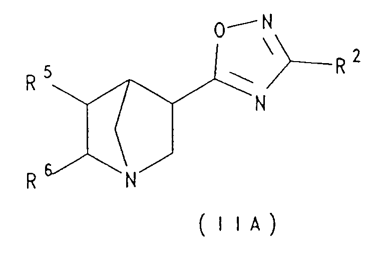 worin R² die in Anspruch 1 gegebene Definition besitzt,
R⁵ für C₁₋₄-Alkyl, Halogen, -NR⁷R⁸, C₁₋₄-Alkoxy, Hydroxy-(C₁₋₄)-alkyl, C₁₋₄-Alkoxy-(C₁₋₄)-alkyl oder Hydroxy steht und R⁶ Wasserstoff, C₁₋₄-Alkyl, C₁₋₄-Alkoxy oder Hydroxy darstellt; und
R⁷ und R⁸ unabhängig für Wasserstoff oder C₁₋₂-Alkyl stehen.

3. Verfahren nach Anspruch 1, bei dem das Oxadiazol durch die Formel IIB 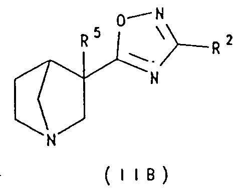 dargestellt wird, worin R² die in Anspruch 1 gegebene Definition besitzt und R⁵ die in Anspruch 2 gegebene Definition besitzt.

4. Verfahren nach Anspruch 1, bei dem das Oxadiazol durch die Formel IIC 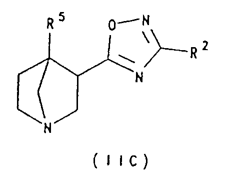 dargestellt wird, worin R² die in Anspruch 1 gegebene Definition besitzt und R⁵ die in Anspruch 2 gegebene Definition besitzt.

5. Verfahren nach einem der Ansprüche 1 bis 4, bei dem R³ Halogen, C₁₋₄-Alkoxy, Carboxy, -NR⁷R⁸, C₂₋₄-Alkyl, C₁₋₄-Alkyl, substituiert mit Hydroxy, oder C₁₋₄-Alkoxy oder Methyl oder Hydroxy in der Position 3, 4 oder 5 darstellt; und R⁴ für Wasserstoff, Halogen,
C₁₋₄-Alkoxy, Hydroxy, Carboxy, -NR⁷R⁸, C₁₋₄-Alkyl oder C₁₋₄-Alkyl, substituiert mit Hydroxy, oder C₁₋₄-Alkoxy steht; oder R³ und R⁴ zusammen Carbonyl darstellen.

6. Verfahren nach einem der Ansprüche 1 bis 5, bei dem R⁴ für Wasserstoff oder Methyl steht; und R³ C₁₋₄-Alkoxy, Halogen, -NR⁷R⁸, Hydroxy-(C₁₋₄)-alkyl oder C₁₋₄-Alkoxymethyl bedeutet.

7. Verfahren nach einem der Ansprüche 1 bis 6, bei dem R³ Methoxy, Fluor, Amino, Methoxymethyl oder Hydroxymethyl bedeutet.

8. Verfahren nach einem der Ansprüche 1 bis 7, bei dem R⁴ für Wasserstoff steht.

9. Verfahren nach Anspruch 1, bei dem das Oxadiazol ausgewählt wird aus:
3-[5-(3-Methyl-1,2,4-oxadiazol)-yl]-1-azabicyclo[2.2.1]heptan-3-ol;
3-[5-(3-Methyl-1,2,4-oxadiazol)-yl]-1-azabicyclo[2.2.1]heptan-5-ol;
3-[5-(3-Methyl-1,2,4-oxadiazol)-yl]-5-ethyl-1-azabicyclo[2.2.1]heptan;
3-[5-(3-Methyl-1,2,4-oxadiazol)-yl]-6-methyl-1-azabicyclo[2.2.1]heptan;
3-[5-(3-Amino-1,2,4-oxadiazol)-yl]-1-azabicyclo[2.2.1]heptan-5-ol;
3-[5-(3-Methyl-1,2,4-oxadiazol)-yl]-5-fluor-1-azabicyclo[2.2.1]heptan;
5-[5-(3-Methyl-1,2,4-oxadiazol)-yl]-3-methoxymethyl-1-azabicyclo[2.2.1]heptan;
5-[5-(3-Methyl-1,2,4-oxadiazol)-yl]-3-methyl-1-azabicyclo[2.2.1]heptan;
3-[5-(3-Methyl-1,2,4-oxadiazol)-yl]-5-hydroxymethyl-1-azabicyclo[2.2.1]heptan;
3-[5-(3-Dimethylamino-1,2,4-oxadiazol)-yl]-1-azabicyclo-[2,.2.1]heptan-5-ol;
3-[5-(3-Dimethylamino-1,2,4-oxadiazol)-yl]-5-methoxy-1-azabicyclo[2.2.1]heptan;
3-[5-(3-Dimethylamino-1,2,4-oxadiazol)-yl]-5-methyl-1-azabicyclo[2.2.1]heptan-5-ol;
3-[5-(3-Dimethylamino-1,2,4-oxadiazol)-yl]-5-amino-1-azabicyclo[2.2.1]heptan und
3-[5-(3-Methyl-1,2,4-oxadiazol)-yl]-4-methyl-1-azabicyclo[2.2.1]heptan;
und Salze und Arzneimittelvorstufen davon

10. Verfahren nach Anspruch 1, bei dem das Oxadiazol ausgewählt wird aus:
3-[5-(3-Methyl-1,2,4-oxadiazol)-yl]-1-azabicyclo[2.2.1]heptan-5-ol;
3-[5-(3-Methyl-1,2,4-oxadiazol)-yl]-1-azabicyclo[2.2.1]heptan-3-ol;
3-[5-(3-Methyl-1,2,4-oxadiazol)-yl]-4-methyl-1-azabicyclo[2.2.1]heptan;
3-[5-(3-Methyl-1,2,4-oxadiazol)-yl]-5-ethyl-1-azabicyclo[2.2.1]heptan;
3-[5-(3-Methyl-1,2,4-oxadiazol)-yl]-6-methyl-1-azabicyclo[2.2.1]heptan;
exo-3-[5-(3-Amino-1,2,4-oxadiazol)-yl]-1-azabicyclo[2.2.1]heptan-5-ol;
3-[5-(3-Amino-1,2,4-oxadiazol)-yl]-5,5-dimethoxy-1-azabicyclo[2.2.1]heptan;
3-[5-(3-Amino-1,2,4-oxadiazol)-yl]-1-azabicyclo[2.2.1]heptan-5-on;
exo-3-[5-(3-Methyl-1,2,4-oxadiazol)-yl]-5-fluoro-1-azabicyclo[2.2.1]heptan;
exo-5-[5-(3-Methyl-1,2,4-oxadiazol)-yl]-3-methoxymethyl-1-azabicyclo[2.2.1]heptan;
exo-5-[5-(3-Methyl-1,2,4-oxadiazol)-yl]-3-methyl-1-azabicyclo[2.2.1]heptan und
exo-3-[5-(3-Methyl-1,2,4-oxadiazol)-yl]-5-hydroxymethyl-1-azabicyclo[2.2.1]heptan
und Salze und Arzneimittelvorstufen davon.

11. Verfahren nach Anspruch 1, bei dem das Oxadiazol ausgewählt wird aus
3-[5-(3-Methyl-1,2,4-oxadiazol)-yl]-1-azabicyclo[2.2.1]heptan-5-ol;
exo-3-[5-(3-Methyl-1,2,4-oxadiazol)-yl]-1-azabicyclo[2.2.1]heptan-3-ol;
und Salze und Arzneimittelvorstufen davon.

12. Verfahren nach Anspruch 1, bei dem das Oxadiazol 3-[5-(3-Methyl-1,2,4-oxadiazol)-yl]-6-methyl-1-azabicyclo[2,2,1]heptan und Salze und Arzneimittelvorstufen davon darstellt.

13. Verfahren zur Herstellung eines pharmazeutischen Präparats, umfassend ein Oxadiazol nach einem der vorgenannten Ansprüche, und sein Vermischen mit einem pharmazeutisch verträglichen Trägerstoff.

14. Verfahren zur Herstellung einer Zwischenstufe der Formel (V) 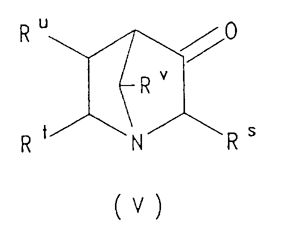 worin R^{s}, R^{t}, R^{u} und R^{v} für Wasserstoff,
Carbonsäureester oder einen Substituenten R³ oder R⁴ nach Anspruch 1 oder eine Gruppe, die dazu umwandelbar ist, stehen, wobei mindestens eines von R^{s}, R^{t}, R^{u} und R^{v} etwas anderes als Wasserstoff bedeutet,
wobei das Verfahren umfaßt Cyclisieren einer Verbindung der Formel IV: 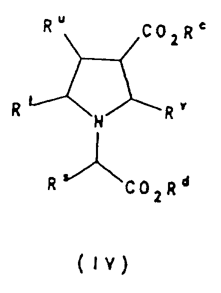 worin R^{s}, R^{t}, R^{u} und R^{v} die oben gegebenen Definitionen besitzen und R^{c} und R^{d} für Kohlenwasserstoffgruppen stehen.

## Revendications (Revendications pour l'(les) Etat(s) contractant(s) suivant(s): AT, BE, CH, DE, FR, GB, IT, LI, LU, NL, SE)

1. Oxadiazole représenté par la formule développée (I) : 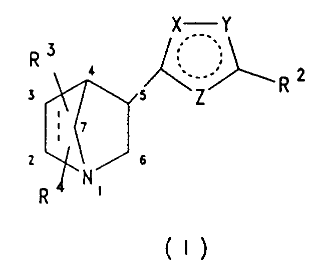 ou un de ses sels ou pro-médicaments ; dans laquelle
l'un d'entre X, Y ou Z représente un atome d'oxygène et les deux autres représentent des atomes d'azote, et le cercle en pointillés représente le caractère aromatique (deux doubles liaisons) ;
R² peut-être un atome d'hydrogène ou d'halogène ou un groupe -CF₃, -OR⁷, -NR⁷R⁸, -NHOR⁷, -NHNH₂, -CN, -CO₂R⁷, -CON⁷R⁸, alcényle en C₂₋₅, alcynyle en C₂₋₅, alkyle en C₁₋₂ ou alkyle en C₁₋₂ substitué par un groupe -OR⁷, -NR⁷R⁸, -SR⁷, -CO₂R⁷, -CONR⁷R⁸ ou un atome d'halogène ;
la ligne discontinue représente une liaison chimique facultative ;
et
les substituants R³ et R⁴ peuvent être présents en position 3-, 4-ou 5- du noyau azabicyclique, y compris le point de rattachement au noyau oxadiazole, et R³ représente un atome d'halogène ou un groupe alcoxy en C₁₋₄, carboxy, -NR⁷R⁸, alkyle en C₁₋₄, alkyle en C₁₋₄ substitué par un groupe hydroxyle ou alcoxy en C₁₋₄, ou hydroxyle ; et R⁴ représente un atome d'hydrogène ou d'halogène ou un groupe alcoxy en C₁₋₄, hydroxyle, carboxy, -NR⁷R⁸, alkyle en C₁₋₄ ou alkyle en C₁₋₄ substitué par un groupe hydroxyle ou alcoxy en C₁₋₄ ; ou R³ et R⁴ représentent conjointement le groupe carbonyle ; et
R⁷ et R⁸ représentent indépendamment un atome d'hydrogène ou un groupe alkyle en C₁₋₂.

2. Oxadiazole selon la revendication 1, représenté par la formule IIA : 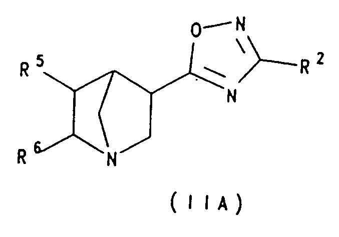 dans laquelle R² est tel que défini dans la revendication 1, R⁵ représente un groupe alkyle en C₁₋₄, halogéno, -NR⁷R⁸, alcoxy en C₁₋₄, hydroxyalkyle en C₁₋₄, (alcoxy en C₁₋₄)alkyle en C₁₋₄ ou hydroxyle ; et R⁶ représente un atome d'hydrogène ou un groupe alkyle en C₁₋₄, alcoxy en C₁₋₄ ou hydroxyle ; et
R⁷ et R⁸ représentent indépendamment un atome d'hydrogène ou un groupe alkyle en C₁₋₂.

3. Oxadiazole selon la revendication 1, représenté par la formule IIB 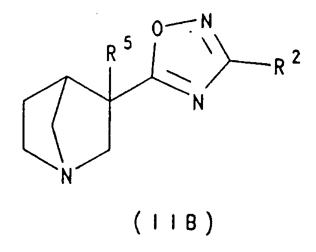 dans laquelle R² est tel que défini dans la revendication 1 et R⁵ est tel que défini dans la revendication 2.

4. Oxadiazole selon la revendication 1, représenté par la formule IIC 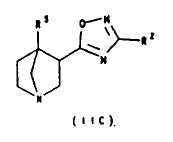 dans laquelle R² est tel que défini dans la revendication 1 et R⁵ est tel que défini dans la revendication 2.

5. Oxadiazole selon l'une quelconque des revendications 1 à 4 dans lequel R³ représente un atome d'halogène ou un groupe alcoxy en C₁₋₄, carboxyle, -NR⁷R⁸, alkyle en C₂₋₄, alkyle en C₁₋₄ substitué par un groupe hydroxyle ou alcoxy en C₁₋₄, ou méthyle ou hydroxyle en position 3-, 4- ou 5- ; et R⁴ représente un atome d'hydrogène ou d'halogène ou un groupe alcoxy en C₁₋₄, hydroxyle, carboxy, -NR⁷R⁸, alkyle en C₁₋₄ ou alkyle en C₁₋₄ substitué par un groupe hydroxyle ou alcoxy en C₁₋₄ ; ou R³ et R⁴ repésentent conjointement le groupe carbonyle.

6. Oxadiazole selon l'une quelconque des revendications 1 à 5, dans lequel R⁴ représente un atome d'hydrogène ou un groupe méthyle ; et R³ représente un groupe alcoxy en C₁₋₄, halogéno, -NR⁷R⁸, hydroxyalkyle en C₁₋₄ ou (alcoxy en C₁₋₄)méthyle.

7. Oxadiazole selon l'une quelconque des revendications 1 à 6, dans lequel R³ représente un groupe méthoxy, fluoro, amino, méthoxyméthyle ou hydroxyméthyle.

8. Oxadiazole selon l'une quelconque des revendications 1 à 7, dans lequel R⁴ représente un atome d'halogène.

9. Composé selon la revendication 1, choisi parmi les suivants :
3-[5-(3-méthyl-1,2,4-oxadiazol)-yl]-1-azabicyclo[2.2.1]heptan-3-ol;
3-[5-(3-méthyl-1,2,4-oxadiazol)-yl]-1-azabicyclo[2.2.1]heptan-5-ol;
3-[5-(3-méthyl-1,2,4-oxadiazol)-yl]-5-éthyl-1-azabicyclo[2.2.1]heptane;
3-[5-(3-méthyl-1,2,4-oxadiazol)-yl]-6-méthyl-1-azabicyclo[2.2.1]-heptane;
3-[5-(3-amino-1,2,4-oxadiazol)-yl]-1-azabicyclo[2.2.1]heptan-5-ol;
3-[5-(3-méthyl-1,2,4-oxadiazol)-yl]-5-fluoro-1-azabicyclo[2.2.1]heptane;
5-[5-(3-méthyl-1,2,4-oxadiazol)-yl]-3-méthoxyméthyl-1-azabicyclo-[2.2.1]heptane;
5-[5-(3-méthyl-1,2,4-oxadiazol)-yl]-3-méthyl-1-azabicyclo[2.2.1]heptane;
3-[5-(3-méthyl-1,2,4-oxadiazol)-yl]-5-hydroxyméthyl-1-azabicyclo-[2.2.1]heptane;
3-[5-(3-diméthylamino-1,2,4-oxadiazol)-yl]-1-azabicyclo[2.2.1]heptan-5-ol:
3-[5-(3-diméthylamino-1,2,4-oxadiazol)-yl]-5-méthoxy-1-azabicyclo[2.2.1]heptane;
3-[5-(3-dimèthylamino-1,2,4-oxadiazol)-yl]-5-méthyl-1-azabicyclo[2.2.1]heptan-5-ol;
3-[5-(3-diméthylamino-1,2,4-oxadiazol)-yl]-5-amino-1-azabicyclo[2.2.1]heptane; and
3-[5-(3-méthyl-1,2,4-oxadiazol)-yl]-4-méthyl-1-azabicyclo-[2.2.1]heptane;
et leurs sels et pro-médicaments.

10. Composé selon la revendication 1, choisi parmi les suivants :
3-[5-(3-méthyl-1,2,4-oxadiazol)-yl]1-azabicyclo-[2.2.1]-heptan-5-ol;
-3-[5-(3-méthyl-1,2,4-oxadiazol)-yl]1-azabicyclo-[2.2.1]-heptan-3-ol;
3-[5-(3-méthyl-1,2,4-oxadiazol)-yl]-4-méthyl-1-azabicyclo-[2.2.1]heptane;
3-[5-(3-méthyl-1,2,4-oxadiazol)-yl]-5-éthyl-1-azabicyclo[2.2.1]-heptane:
-3-[5-(3-méthyl-1,2,4-oxadiazol)-yl]-6-méthyl-1-azabicyclo[2.2.1]heptane;
Exo-3-[5-(3-amino-1,2,4-oxadiazol)-yl]-1-azabicyclo[2.2.1]heptan-5-ol;
3-[5-(3-amino-1,2,4-oxadiazol)-yl]-5,5-diméthoxy-1-azabicyclo[2.2.1]heptane;
3-[5-(3-amino-1,2,4-oxadiazol)-yl]-1-azabicyclo[2.2.1]heptan-5-one;
Exo-3-[5-(3-méthyl-1,2,4-oxadiazol)-yl]-5-fluoro-1-azabicyclo[2.2.1]heptane;
Exo-5-[5-(3-méthyl-1,2,4-oxadiazol)-yl]-3-méthoxyméthyl-1-azabicyclo[2.2.1]heptane;
Exo-5-[5-(3-méthyl-1,2,4-ozadiazol)-yl]-3-méthyl-1-azabicyclo[2.2.1]heptane; and
Exo-3-[5-(3-méthyl-1,2,4-oxadiazol)-yl]-5-hydroxyméthyl-1-azabicyclo[2.2.1]heptane;
et leurs sels et pro-médicaments.

11. Composé selon la revendication 1, choisi parmi les suivants :
3-[5-(3-méthyl-1,2,4-oxadiazol)yl]-1-azabicyclo-[2.2.1]-heptan-5-ol;
Exo-3-[5-(3-méthyl-1,2,4-oxadiazol)yl]-1-azabicyclo[2.2.1]-heptan-3-ol;
et leurs sels et pro-médicaments.

12. 3-(5-(3-méthyl-1,2,4-oxadiazol)yl)-6-méthyl-1-azabicyclo(2.2.1)heptane et ses sels et pro-médicaments.

13. Composition pharmaceutique qui comprend un composé selon l'une quelconque de revendications précédentes, en association avec un véhicule pharmaceutiquement acceptable.

14. Composition pharmaceutique selon la revendication 13, qui comprend en outre un antagoniste cholinergique périphérique.

15. Utilisation d'un composé selon l'une quelconque des revendications 1 à 12, pour la préparation d'un médicament pour le traitement des troubles neurologiques et mentaux pour le traitement des douleurs graves.

16. Procédé de préparation d'un composé selon l'une quelconque des revendications 1 à 12, qui comprend la réaction d'un dérivé réactif d'un acide carboxylique de formule R^{a}-CO₂H avec soit un composé de formule IIIA, soit un composé de formule IIIB ou un de leurs sels : 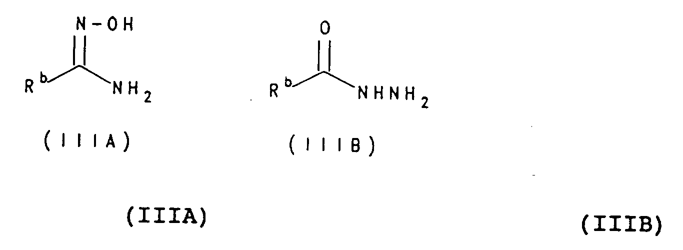 dans lesquelles l'un d'entre R^{a} et R^{b} est un noyau 1-azabicyclo(2.2-.1)heptane substitué par un ou plusieurs substituants R³ et R⁴ tels que définis dans la revendication 1, et l'autre étant un groupe de faible lipophilie, comme défini dans la revendication 1.

17. Intermédiaire de formule (V) 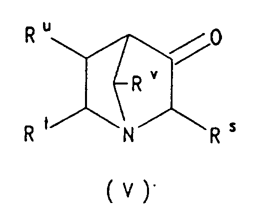 dans laquelle R^{s}, R^{t}, R^{u} et R^{v} représentent un atome d'hydrogène, un ester d'acide carboxylique ou un substituant R³ ou R⁴ tels que définis dans la revendication 1, ou un groupe qui est convertible en ceux-ci au moins l'un d'entre R^{s}, R^{t}, R^{u} et R^{v} étant différent d'un atome d'hydrogène.

18. Procédé de préparation d'un composé selon la revendication 17, qui comprend la cyclisation d'un composé de formule (IV) : 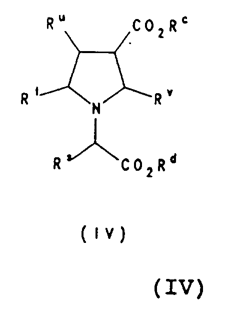 dans laquelle R^{s}, R^{t}, R^{u} et R^{v} sont tels que définis dans la revendication 17, et R^{c} et R^{d} représentent des groupes hydrocarbonés.

## Revendications (Revendications pour l'(les) Etat(s) contractant(s) suivant(s): ES, GR)

1. Procédé de préparation d'un oxadiazole représenté par la formule développée (I) : 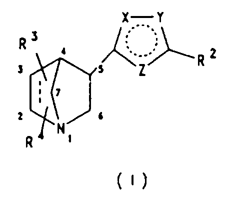 ou d'un de ses sels ou pro-médicaments ; dans laquelle
l'un d'entre X, Y ou Z représente un atome d'oxygène et les deux autres représentent des atomes d'azote, et le cercle en pointillés représente le caractère aromatique (deux doubles liaisons) ;
R² peut-être un atome d'hydrogène ou d'halogène ou un groupe -CF₃, -OR⁷, -NR⁷R⁸, -NHOR⁷, -NHNH₂, -CN, -CO₂R⁷, -CON⁷R⁸, alcényle en C₂₋₅, alcynyle en C₂₋₅, alkyle en C₁₋₂ ou alkyle en C₁₋₂ substitué par un groupe -OR⁷, -NR⁷R⁸, -SR⁷, -CO₂R⁷, -CONR⁷R⁸ ou un atome d'halogène ;
la ligne discontinue représente une liaison chimique facultative ;
et
les substituants R³ et R⁴ peuvent être présents en position 3-, 4-ou 5- du noyau azabicyclique, y compris le point de rattachement au noyau oxadiazole, et R³ représente un atome d'halogène ou un groupe alcoxy en C₁₋₄, carboxy, -NR⁷R⁸, alkyle en C₁₋₄, alkyle en C₁₋₄ substitué par un groupe hydroxyle ou alcoxy en C₁₋₄, ou hydroxyle ; et R⁴ représente un atome d'hydrogène ou d'halogène ou un groupe alcoxy en C₁₋₄, hydroxyle, carboxy, -NR⁷R⁸, alkyle en C₁₋₄ ou alkyle en C₁₋₄ substitué par un groupe hydroxyle ou alcoxy en C₁₋₄ ; ou R³ et R⁴ représentent conjointement le groupe carbonyle ; et
R⁷ et R⁸ représentent indépendamment un atome d'hydrogène ou un groupe alkyle en C₁₋₂ ;
ce procédé comprenant la réaction d'un dérivé réactif d'un acide carboxylique de formule R^{a}-CO₂H avec soit un composé de formule IIIA soit un composé de formule IIIB ou un de leurs sels : 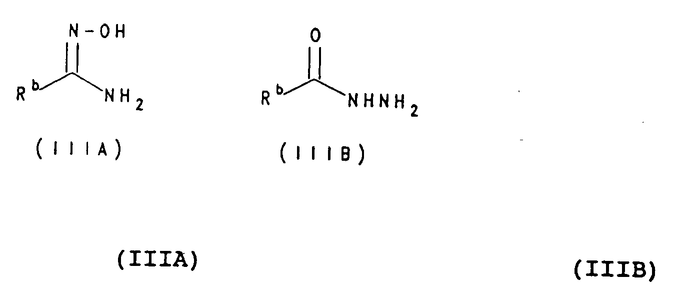 où l'un d'entre R^{a} et R^{b} est un noyau 1-azabicyclo(2.2.1)heptane substitué par un ou plusieurs substituants R³ et R⁴ tels que définis ci-dessus, et l'autre étant un groupe de faible lipophilie tel défini ci-dessus.

2. Procédé selon la revendication 1, dans lequel l'oxadiazole est représenté par la formule IIA : 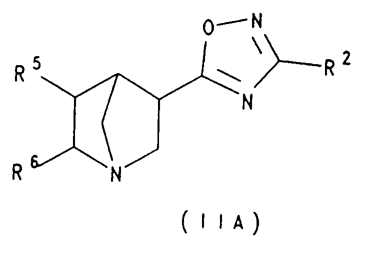 dans laquelle R² est tel que défini dans la revendication 1, R⁵ représente un groupe alkyle en C₁₋₄, halogéno, -NR⁷R⁸, alcoxy en C₁₋₄, hydroxyalkyle en C₁₋₄, (alcoxy en C₁₋₄)alkyle en C₁₋₄ ou hydroxyle ; et R⁶ représente un atome d'hydrogène ou un groupe alkyle en C₁₋₄, alcoxy en C₁₋₄ ou hydroxyle ; et
R⁷ et R⁸ représentent indépendamment un atome d'hydrogène ou un groupe alkyle en C₁₋₂.

3. Procédé selon la revendication 1, dans lequel l'oxadiazole est représenté par la formule IIB 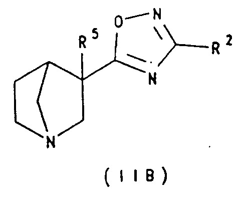 dans laquelle R² est tel que défini dans la revendication 1 et R⁵ est tel que défini dans la revendication 2.

4. Procédé selon la revendication 1, dans lequel l'oxadiazole est représenté par la formule IIC 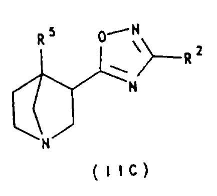 dans laquelle R² est tel que défini dans la revendication 1 et R⁵ est tel que défini dans la revendication 2.

5. Procédé selon l'une quelconque des revendications 1 à 4, dans lequel R³ représente un atome d'halogène ou un groupe alcoxy en C₁₋₄, carboxyle, -NR⁷R⁸, alkyle en C₂₋₄, alkyle en C₁₋₄ substitué par un groupe hydroxyle ou alcoxy en C₁₋₄, ou méthyle ou hydroxyle en position 3-, 4- ou 5- ; et R⁴ représente un atome d'hydrogène ou d'halogène ou un groupe alcoxy en C₁₋₄, hydroxyle, carboxy, -NR⁷R⁸, alkyle en C₁₋₄ ou alkyle en C₁₋₄ substitué par un groupe hydroxyle ou alcoxy en C₁₋₄ ; ou R³ et R⁴ repésentent conjointement le groupe carbonyle.

6. Procédé selon l'une quelconque des revendications 1 à 5, dans lequel R⁴ représente un atome d'hydrogène ou un groupe méthyle ; et R³ représente un groupe alcoxy en C₁₋₄, halogéno, -NR⁷R⁸, hydroxyalkyle en C₁₋₄ ou (alcoxy en C₁₋₄)méthyle.

7. Procédé selon l'une quelconque des revendications 1 à 6, dans lequel R³ représente un groupe méthoxy, fluoro, amino, méthoxyméthyle ou hydroxyméthyle.

8. Procédé selon l'une quelconque des revendications 1 à 7, dans lequel R⁴ représente un atome d'halogène.

9. Procédé selon la revendication 1, dans lequel l'oxadiazole est choisi parmi les suivants :
3-[5-(3-méthyl)-1,2,4-oxadiazol)-yl]-1-azabicyclo[2.2.1]heptan-3-ol;
3-[5-(3-méthyl-1,2,4-oxadiazol)-yl]-1-azabicyclo[2.2.1]heptan-5-ol;
3-[5-(3-méthyl-1,2,4-oxadiazol)-yl]-5-éthyl-1-azabicyclo[2.2.1]heptane;
3-[5-(3-méthyl-1,2,4-oxadiazol)-yl]-6-méthyl-1-azabicyclo[2.2.1]-heptane;
3-[5-(3-amino-1,2,4-oxadiazol)-yl]-1-azabicyclo[2.2.1]heptan-5-ol;
3-[5-(3-méthyl-1,2,4-oxadiazol)-yl]-5-fluoro-1-azabicyclo[2.2.1]heptane;
5-[5-(3-méthyl)-1,2,4-oxadiazol)-yl]-3-méthoxyméthyl-1-azabicyclo-[2.2.1]heptane;
5-[5-(3-méthyl-1,2,4-oxadiazol)-yl]-3-méthyl-1-azabicyclo[2.2.1]heptane;
3-[5-(3-méthyl-1,2,4-oxadiazol)-yl]-5-hydroxyméthyl-1-azabicyclo-[2.2.1]heptane;
3-[5-(3-diméthylamino-1,2,4-oxadiazol)-yl]-1-azabicyclo[2.2.1]heptan-5-ol;
3-[5-(3-diméthylamino-1,2,4-oxadiazol)-yl]-5-méthoxy-1-azabicyclo[2.2.1]heptane;
3-[5-(3-diméthylamino-1,2,4-oxadiazol)-yl]-5-méthyl-1-azabicyclo[2.2.1]heptane-5-ol;
3-[5-(3-diméthylamino-1,2,4-oxadiazol)-yl]-5-amino-1-azabicyclo[2.2.1]heptane;
3-[5-(3-méthyl-1,2,4-oxadiazol)-yl]-4-méthyl-1-azabicyclo-[2.2.1]heptane;
et leurs sels et pro-médicaments.

10. Procédé selon la revendication 1, dans lequel l'oxadiazole est choisi parmi les suivants :
3-[5-(3-méthyl-1,2,4-oxadiazol)-yl]1-azabicyclo-[2.2.1]-heptan-5-ol;
-3-[5-(3-méthyl-1,2,4-oxadiazol)-yl]1-azabicyclo-[2.2.1]-heptan-3-ol;
3-[5-(3-méthyl-1,2,4-oxadiazol)-yl]-4-méthyl-1-azabicyclo-[2.2.1]heptane;
3-[5-(3-méthyl-1,2,4-oxadiazol)-yl]-5-éthyl-1-azabicyclo[2.2.1]-heptane;
-3-[5-(3-méthyl-1,2,4-oxadiazol)-yl]-6-méthyl-1-azabicyclo[2.2.1]heptane;
Exo-3-[5-(3-amino-1,2,4-oxadiazol)-yl]-1-azabicyclo[2.2.1]heptan-5-ol;
3-[5-(3-amino-1,2,4-oxadiazol)-yl]-5,5-diméthoxy-1-azabicyclo[2.2.1]heptane;
3-[5-(3-amino-1,2,4-oxadiazol)-yl]-1-azabicyclo[2.2.1]heptan-5-one;
Exo-3-[5-(3-méthyl-1,2,4-oxadiazol)-yl]-5-fluoro-1-azabicyclo[2.2.1]heptane;
Exo-5-[5-(3-méthyl-1,2,4-oxadiazol)-yl]-3-méthoxyméthyl-1-azabicyclo[2.2.1]heptane;
Exo-5-[5-(3-méthyl-1,2,4-ozadiazol)-yl]-3-méthyl-1-azabicyclo[2.2.1]heptane; and
Exo-3-[5-(3-méthyl-1,2,4-oxadiazol)-yl]-5-hydroxyméthyl-1-azabicyclo[2.2.1]heptane;
et leurs sels et pro-médicaments.

11. Procédé selon la revendication 1, dans lequel l'oxadiazole est choisi parmi les suivants :
le 3-(5-(3-méthyl-1,2,4-oxadiazol)yl)-1-azabicyclo(2.2.1)heptan-5-ol ; l'exo-3-(5-(3-méthyl-1,2,4-oxadiazol)yl)-1-azabicyclo(2.2.1)-heptan-3-ol ;
et leurs sels et pro-médicaments.

12. Procédé selon la revendication 1, dans lequel l'oxadiazole est le 3-(5-(3-méthyl-1,2,4-oxadiazol)yl)-6-méthyl-1-azabicyclo(2.2.1)heptane ou un de ses sels et pro-médicaments.

13. Procédé de préparation d'une composition pharmaceutique qui comprend la préparation d'un oxadiazole selon l'une quelconque des revendications précédentes et sa mise en association avec un véhicule pharmaceutiquement acceptable.

14. Procédé de préparation d'un intermédiaire de formule (V) 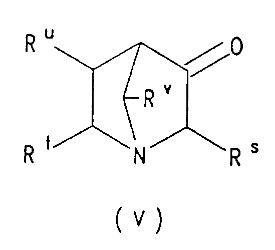 dans laquelle R^{s}, R^{t}, R^{u} et R^{v} représentent un atome d'hydrogène, un groupe ester d'acide carboxylique ou un substituant R³ ou R⁴ tels que définis dans la revendication 1, ou un groupe qui est convertible en ceux-ci, au moins l'un d'entre R^{s}, R^{t}, R^{u} et R^{v} étant différent d'un atome d'hydrogène, qui comprend la cyclisation d'un composé de formule (IV) : 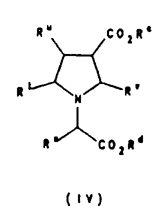 dans laquelle R^{s}, R^{t}, R^{u} et R^{v} sont tels que définis ci-dessus, et R^{c} et R^{d} représentent des groupes hydrocarbones.
